(19) Europäisches Patentamt
European Patent Office
Office européen des brevets

(11) Numéro de publication : **0 292 350 B1**

# (12) FASCICULE DE BREVET EUROPEEN

(45) Date de publication du fascicule du brevet : **21.08.91 Bulletin 91/34**

(51) Int. Cl.$^5$ : **C07C 403/06**, C07F 7/00

(21) Numéro de dépôt : **88401099.2**

(22) Date de dépôt : **05.05.88**

(54) **Procédé de synthèse de la vitamine A et de certains de ses dérivés.**

(30) Priorité : **21.05.87 FR 8707124**

(43) Date de publication de la demande : **23.11.88 Bulletin 88/47**

(45) Mention de la délivrance du brevet : **21.08.91 Bulletin 91/34**

(84) Etats contractants désignés : **AT BE CH DE ES GB GR IT LI NL SE**

(56) Documents cités :
**BE-A- 544 240**
**US-A- 2 577 538**
**JOURNAL OF THE CHEMICAL SOCIETY, vol. 104, 1982, pages 5807-5808, American Chemical Society; H.M. WALBORSKY et al.: "Titanium-induced reductive elimination. Syntheses of 1,3-dienes1"**
**ACCOUNTS OF CHEMICAL RESEARCH, vol. 7, no. 9, septembre 1974, pages 281-286; J.E. Mc MURRY: "Organic chemistry of low-valent Titanium"**

(56) Documents cités :
**HELVETICA CHIMICA ACTA, vol. 50, fasciculus 5, no. 126, 1967, pages 1243-1248; M. VECCHI et al.: "Gas-chromatographische und massenspektrometrische Untersuchung der Trimethylsilyläther von Vitamin A und einigen seiner Isomeren"**

(73) Titulaire : **L'OREAL**
**14, Rue Royale**
**F-75008 Paris (FR)**

(72) Inventeur : **Solladie, Guy**
**4, rue d'Oslo**
**F-67000 Strasbourg (FR)**
Inventeur : **Forestier, Serge**
**14, Hameau des Lauriers**
**F-77410 Claye-Souilly (FR)**
Inventeur : **Lang, Gérard**
**44, avenue Lacour**
**F-95210 Saint-Gratien (FR)**

(74) Mandataire : **Peuscet, Jacques et al**
**Cabinet Peuscet 68, rue d'Hauteville**
**F-75010 Paris (FR)**



Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention est relative à un procédé de synthèse permettant d'obtenir, d'une part, des composés tout-trans pris dans le groupe formé par la vitamine A et ses éthers, le rétinal et l'acide rétinoïque, et, d'autre part, leurs isomères 13-cis.

Dans le brevet japonais n° 3678/1964, on a décrit la réduction, par l'hydrure d'aluminium et de lithium, en solution dans l'éther éthylique, du composé de formule

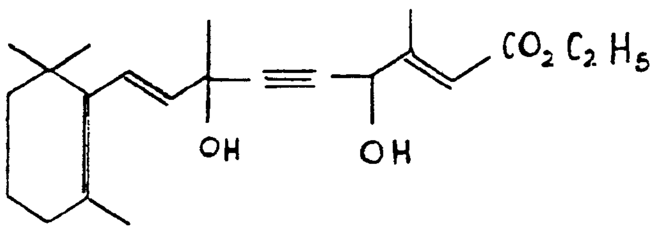

en vitamine A de formule

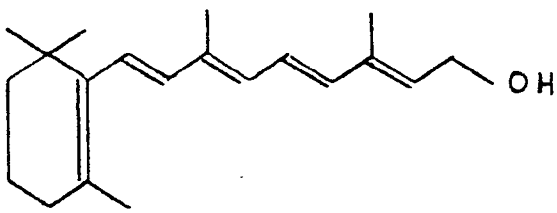

Le rendement de cette réaction serait de 70%. En réalité le rendement obtenu est de 20% en une vitamine A constituée de plusieurs isomères. Ce résultat est en accord avec un travail récent [J. Am. Chem. Soc. 1986, (108), 1338], qui montre que, sur un substrat voisin, la réduction au moyen de cet hydrure conduit à 75% de dérivé tout-trans et 25% d'isomère cis.

S.M. MAKIN [Russian Chem. Revs. 38, 237, (1969) et Zhur. Org. Khim. 2,1586 (1966)] décrit également la réduction d'un diol acétylénique en un dérivé du rétinol sans indication de rendement.

La voie de synthèse explorée par A.D. BROEK et J. LUGTENBURG (Rec. Trav. Chim. Pays-Bas 1980, 363) utilise un organomagnésien acétylénique et conduit à l'obtention d'un mélange tout-trans, 9-cis et 13-cis. Cette voie ne permet donc pas d'atteindre une quelconque stéréospécifité.

Enfin, parmi les méthodes n'utilisant pas de composés acétyléniques, on peut citer les travaux de M. R. FRANSTEN et autres (Rec. Trav. Chim. Pays-Bas, 1980, 384), qui conduisent à l'obtention d'un mélange d'isomères tout-trans et 11-cis.

L'un des buts de la présente invention est de fournir un procédé de synthèse d'un composé précurseur de la vitamine A, tel qu'un éther, qui permet d'obtenir soit l'isomère tout-trans soit l'isomère 13-cis de la Vitamine A, de façon stéréospécifique.

Un autre but de l'invention est de fournir un tel procédé qui permet de produire ce dérivé avec un rendement industriellement intéressant.

Ces buts ainsi que d'autres, qui apparaîtront par la suite, sont atteints par un procédé de synthèse, d'une part, de composés tout-trans pris dans le groupe formé par la vitamine A et ses éthers, le rétinal et l'acide rétinoïque et, d'autre part, de leurs isomères 13-cis, qui est caractérisé par le fait que l'on réalise une réduction stéréospécifique des deux groupes hydroxyles d'un éther-diol précurseur de la vitamine A par un mélange (trichlorure de titane, hydrure d'aluminium et de lithium), en milieu solvant anhydre, à une température comprise entre 5°C et 40°C environ et qu'éventuellement, on transforme l'éther obtenu en vitamine A, rétinal ou acide rétinoïque.

Le milieu solvant est préférentiellement constitué par au moins un éther tel que l'éther diéthylique, le tétrahydrofuranne, le dioxane et leurs mélanges.

Avantageusement, on réduit l'éther-diol à une température d'environ 20°C.

De préférence, on fait réagir 0,8 équivalent d'éther-diol précurseur de la vitamine A avec 2 équivalents de trichlorure de titane et 1 équivalent d'hydrure d'aluminium et de lithium.

Le mélange de trichlorure de titane et d'hydrure d'aluminium et de lithium ($TiCl_3/LiAlH_4$) a été utilisé pour

la première fois par Mc MURRY [Acc. Chem. Res, (1974), 7, 281], pour le couplage réductif de composés carbonylés en oléfines selon le schéma réactionnel :

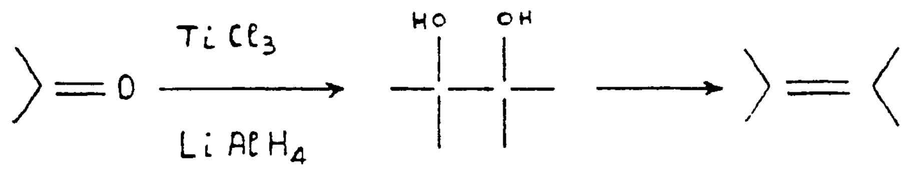


Par la suite, H.M. WALBORSKY [J. Am. Chem. Soc. (1982), 104, 5807] a utilisé ce mélange pour préparer des diènes 1,3 selon le schéma réactionnel :

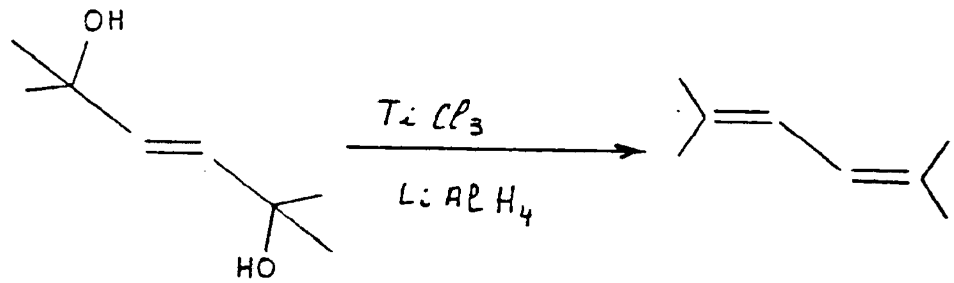


Selon l'invention, on utilise ce mélange réactif pour réduire des diols ayant des parties insaturées de part et d'autre des groupes hydroxyles sans modifier la stéréochimie de la molécule, ce qui est tout à fait inattendu.

De préférence, l'éther-diol précurseur de la vitamine A est choisi dans le groupe constitué par :

— l'éther-diol tout-trans de formule (VII) :

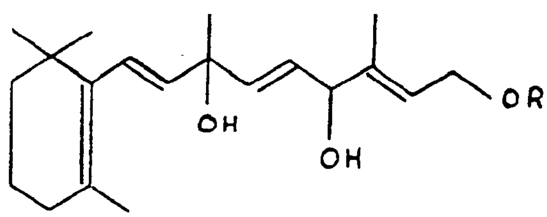

(VII)

formule dans laquelle R est un reste alkyle en $C_1$-$C_6$, un reste aralkyle éventuellement substitué par un ou plusieurs alcoxy, tels que méthoxy-4' benzyle et diméthoxy-3', 4' benzyle, ou un reste

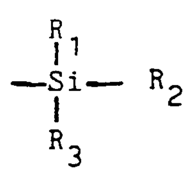

dans lequel $R_1$, $R_2$ et $R_3$, identiques ou différents, représentent un reste alkyle en $C_1$-$C_6$, linéaire ou ramifié, ou un reste aryle éventuellement substitué, tels que le t-butyldiméthylsilane, le t-butyldiphénylsilane ou le triéthylsilane.

— l'éther-diol 10-trans de formule (VIII'a) :

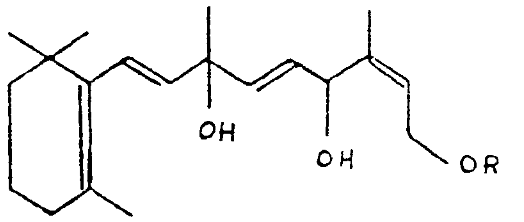

(VIII'a)

et

— l'éther-diol 10-cis de formule (VIII'b) :

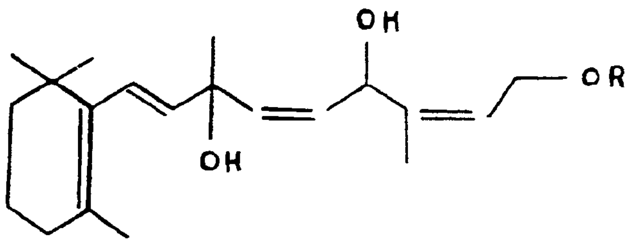

(VIII'b)

formules selon lesquelles R a les significations indiquées ci-dessus.

Selon une première variante d'un premier mode de réalisation de l'invention, on obtient l'éther-diol tout-trans, précurseur de la vitamine A, de formule (VII) par éthérification de la fonction alcool terminal du composé de formule (VIa) :

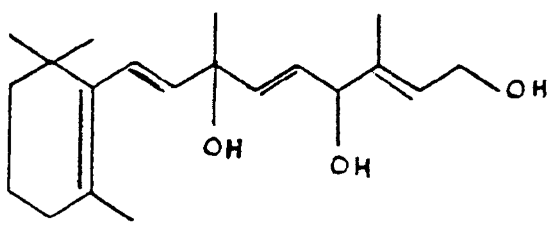

(VI a)

par un reste alkyle en $C_1$-$C_6$, un reste aralkyle éventuellement substitué par un ou plusieurs groupements alcoxy, notamment le méthoxy-4' benzyle et le diméthoxy 3',4' benzyle, ou un reste

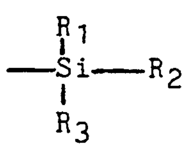

où $R_1$, $R_2$, $R_3$ ont les significations ci-dessus indiquées, notamment le reste t-butyldiméthylsilane, en présence d'imidazole, à température ambiante, suivie d'une extraction à l'éther. Avantageusement, l'extraction à l'éther est suivie par un lavage avec une solution saturée de chlorure d'ammonium, un séchage et une évaporation.

De préférence, pour obtenir le composé de formule (VIa), on fait réagir un ester acétylénique d'éthynyl-β-ionol de formule (V) :

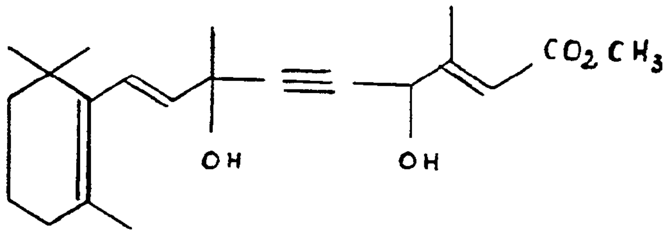

(V)

avec de l'hydrure d'aluminium et de lithium ; après traitement en milieu acide dilué, on extrait la phase aqueuse à l'éther, et on purifie la phase organique, qui contient le composé de formule (VIa).

Avantageusement, pour obtenir le composé de formule (V), on fait réagir le composé connu éthynyl-β-ionol de formule (III) :

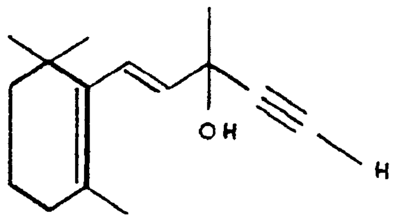

(III)

avec du bromure d'éthylmagnésium puis avec du γ-oxo-sénécioate de méthyle de formule (IV) :

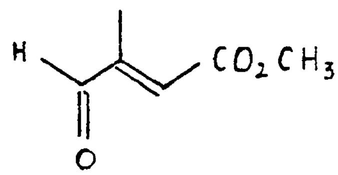

(IV)

Après traitement on extrait à l'éther le composé de formule (V).

Selon une deuxième variante du premier mode de réalisation, on obtient l'éther-diol 10-cis, précurseur de la vitamine A, de formule (VIII'b) par éthérification de la fonction alcool terminal du composé de formule (VIb):

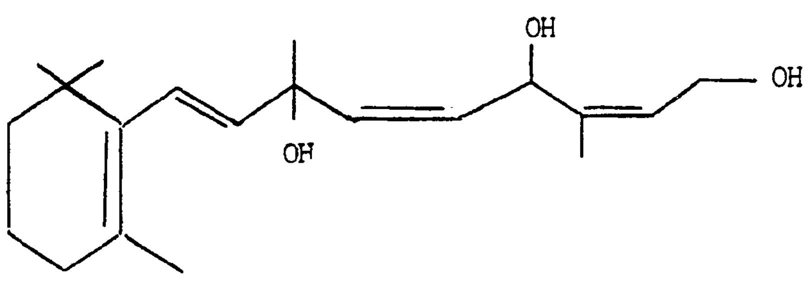

(VIb)

par un reste alkyle en $C_1$-$C_6$, un reste aralkyle éventuellement substitué par un ou plusieurs groupements alcoxy, notamment le méthoxy-4′ benzyle et le diméthoxy-3′, 4′ benzyle, ou un reste Si-$R_1R_2R_3$ où $R_1$, $R_2$, $R_3$ ont les significations ci-dessus indiquées, notamment le reste triéthylsilane, en présence d'imidazole, à température ambiante, suivie d'une extraction à l'éther.

De préférence, pour obtenir le composé de formule (VIb), on réduit le composé de formule (VI') :

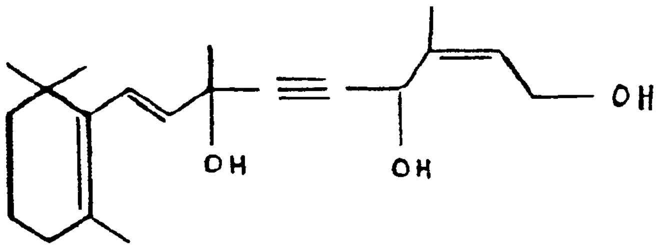

(VI')

par de l'hydrogène, en présence du catalyseur de LINDLAR.

Selon une première variante d'un second mode de réalisation de la présente invention, on obtient l'éther-diol 10-cis, précurseur de la vitamine A, de formule (VIII'b) par réduction par l'hydrogène, en présence du catalyseur de LINDLAR, du composé de formule (VII') :

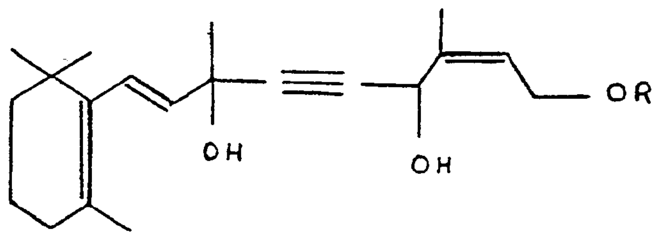

(VII')

dans laquelle R a les mêmes significations que celles ci-dessus indiquées.

Selon une seconde variante de ce second mode de réalisation, on obtient l'éther-diol 10-trans, précurseur de la vitamine A, de formule (VIII'a) par réduction à l'hydrure d'aluminium et de lithium, du composé de formule (VII') ci-dessus indiquée, et extraction à l'éther de la phase organique contenant le composé de formule (VIII'a).

D'après ce second mode de réalisation, on obtient avantageusement le composé de formule (VII') en éthérifiant le composé de formule (VI')

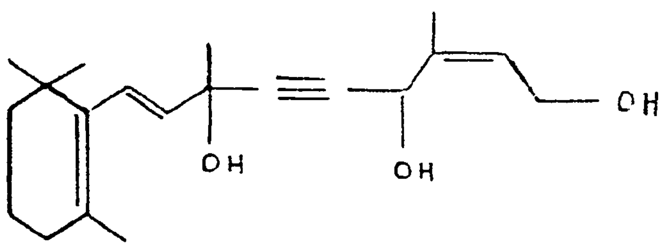

(VI')

par un reste alkyle en $C_1$-$C_6$, un reste aralkyle éventuellement substitué par un ou plusieurs groupements alcoxy, notamment le reste de méthoxy-4' benzyle ou de diméthoxy-3',4' benzyle, ou le reste

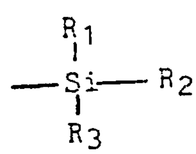

où $R_1$, $R_2$, $R_3$ ont les significations ci-dessus données, tel que le reste t-butyldiphénylsilane, en présence d'imidazole, à température ambiante, cette éthérification étant suivie d'une extraction à l'éther. De préférence, l'extraction à l'éther est suivie par un lavage avec une solution saturée de chlorure d'ammonium, un séchage et une évaporation.

Avantageusement, on obtient le composé de formule (VI') en faisant réagir un composé de formule (V')

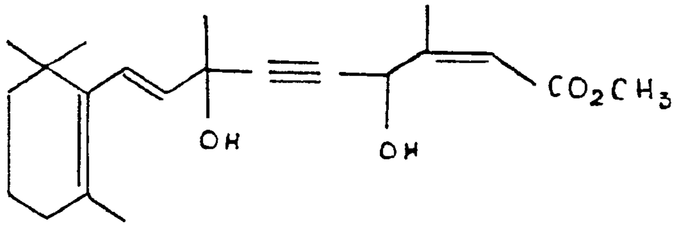

(V')

avec de l'hydrure de diisobutylaluminium, puis en effectuant une extraction de la phase organique, qui contient le composé de formule (VI').

Pour obtenir le composé de formule (V'), on fait, de préférence réagir le composé connu éthynyl-β-ionol de formule (III) avec du bromure d'éthylmagnésium puis avec un composé de formule (IV')

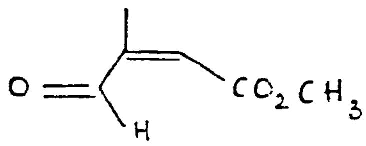

(IV')

que l'on prépare en faisant réagir le buténolide connu de formule

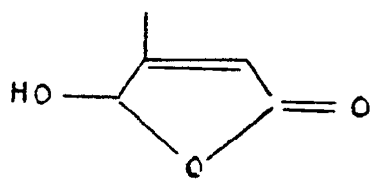

avec de l'hydrure de sodium, puis avec du fluoroborate de triméthyl-oxonium.

Le procédé selon la présente invention permet d'obtenir soit par la première variante du premier mode de réalisation, un éther tout-trans de la vitamine A de formule (VIII) :

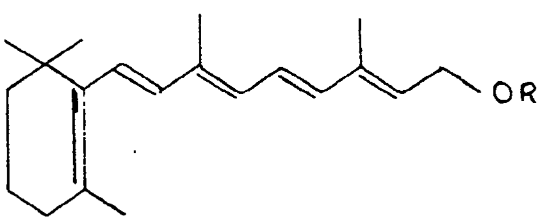

(VIII)

formule dans laquelle R a les significations ci-dessus indiquées, soit par la seconde variante du premier mode de réalisation ou par l'une ou l'autre des deux variantes du second mode de réalisation, un éther 13-cis de la vitamine A de fomule (IX') :

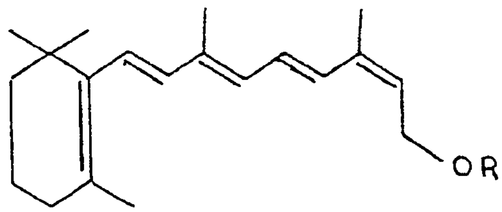

(IX')

dans laquelle R a les significations ci-dessus indiquées.

A partir de ces éthers, il est possible d'obtenir la vitamine A tout-trans, le rétinal tout-trans, l'acide rétinoïque tout-trans, ainsi que leurs isomères 13-cis.

Pour mieux faire comprendre la présente invention, on va en décrire maintenant plusieurs exemples de réalisation.

## Exemple 1

Préparation de l'éther tout-trans de la vitamine A de formule (VIII).

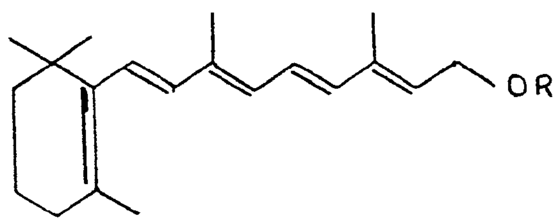

(VIII)

R étant un reste

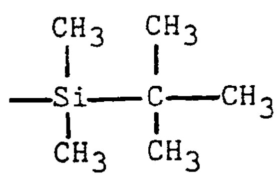

(t-butyldiméthylsilane)

a) Préparation de l'éthynyl-β-ionol de formule (III)

Ce composé connu est obtenu selon le mode préparatoire décrit par A.D. BROEK et J. LUGTENBURG dans Rec. Trav. Chim. Pays-Bas, 1980, 363 :

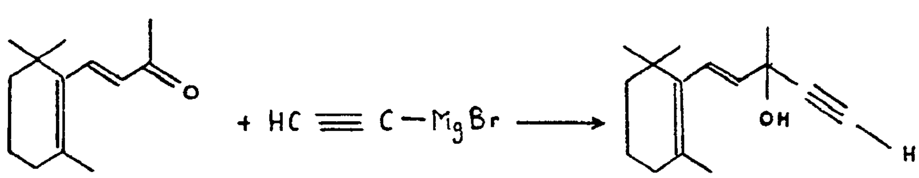

(I) (II) (III)

en faisant réagir la β-ionone (I) avec du bromure d'éthynylmagnésium (II).

Le rendement de cette réaction est de 80% environ.

b) Préparation de l'ester acétylénique éthynyl-β-ionol (V)

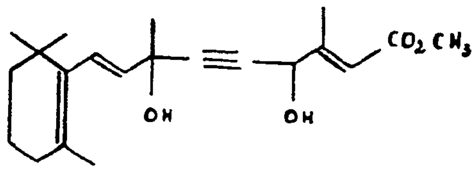

(V)

A une solution de 6,54 g (30 mmoles) d'éthynyl-β-ionol de formule (III) dans 50 ml de tétrahydrofuranne anhydre est ajoutée une solution de bromure d'éthylmagnésium, préparée à partir de 1,75 g de Mg et 7,85 g de bromure d'éthyle (72 mmoles) dans 70 ml de tétrahydrofuranne. Après avoir laissé pendant une heure à température ambiante, on ajoute goutte à goutte 4,69 g (33 moles) de γ-oxo sénécioate de méthyle de formule (IV) en solution dans 30 ml de tétrahydrofuranne anhydre ; ledit composé de formule (IV) a été obtenu par le procédé décrit par K. SISIDO et autres dans J. Am. Chem. Soc. 1960, (82), 2286. Après cette addition, la solution devient petit à petit limpide et jaune vert. L'avancement de la réaction peut être suivi par chromatographie sur couche mince de silice (éluant : acétate d'éthyle/n-hexane dans le rapport volumique 30/70, Rf = 0,25).

Après une heure à température ambiante, on verse le mélange réactionnel dans 200 ml d'une solution saturée de $NH_4Cl$ refroidie à 0°C.

Le produit extrait à l'éther et séché sur sulfate de sodium est ensuite purifié par chromatographie (éluant: acétate d'éthyle/n-hexane dans le rapport volumique 30/70).

Le rendement en ester acétylénique d'éthynyl-β- ionol (V) isolé après chromatographie est de 80% environ.

L'analyse par résonance magnétique nucléaire (200 MHz, $CDCl_3$) a donné les résultats suivants :

δ : 0,98 (s, 6H, $CH_3$ en 1) ; 1,42-1,64 (m, 4H, $CH_2$ en 2 et 3) ; 1,60 (s, 3H, $CH_3$ en 9) ; 1,66 (s, 3H, $CH_3$ en 5) ; 1,98 (t, 2H, $CH_2$ en 4, J = 6 Hz) ; 2,24 (s, 3H, $CH_3$ en 13) ; 3,72 (s, 3H, $OCH_3$) ; 4,89 (s large, 1H, H en 12) ; 5,95 (AB, 2H, J = 16 Hz, Δν = 159 Hz, H en 7 et 8) ; 6,12 (s large, 1 H, H en 14).

c) Préparation du triol de formule (VIa)

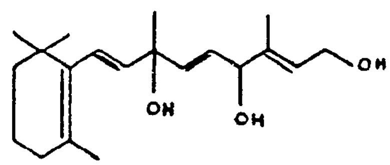

(VIa)

A une solution de 1,5 g de l'ester acétylénique de formule (V) obtenu dans l'étape précédente dans 40 ml de tétrahydrofuranne anhydre à −20°C, on ajoute 2 équivalents dosés de Li $AlH_4$ (330 mg). Après l'addition, le mélange est chauffé à 30°C pendant 2 heures. On ajoute quelques gouttes d'acétate d'éthyle puis quelques gouttes d'eau et enfin 40 ml d'une solution aqueuse d'HCl à 5% en poids.

Les phases sont séparées et la phase aqueuse extraite à l'éther (4 × 20 ml). Les phases organiques sont ensuite lavées par 50 ml d'eau saturée de NaCl et séchées sur $SO_4Mg$.

Le produit est ensuite purifié par chromatographie (éluant : acétate d'éthyle/n-hexane dans le rapport volumique 80/20 ; Rf = 0,2). Le rendement en produit (VIa) isolé est d'environ 80%.

Le produit est une huile incolore, dont l'analyse par résonance magnétique nucléaire (200 MHz, $CDCl_3$) a donné les résultats suivants :

δ : 0,97 (s, 6H, $CH_3$ en 1) ; 1,43 (s, 3H, $CH_3$ en 9) ; 1,46-1,63 (m, 4H, $CH_2$ en 2 et 3) ; 1,65 (s, 6H, $CH_3$ en 5 et 13) ; 1,89-2,00 (m, $CH_2$ en 4) ; 4,20 (d, 2H, J = 7 Hz, $CH_2$ en 15) ; 4,56 (d, 1H, J = 6 Hz, H en 12) ; 5,79 (AB, 2H, J = 16 Hz, Δν = 107 Hz, $H_7$ et $H_8$) ; 5,65-5,94 (système à 12 raies, 3H, $H_{10}$, $H_{11}$, $H_{14}$).

d) Préparation de l'éther-diol, précurseur de la vitamine A, de formule (VII) :

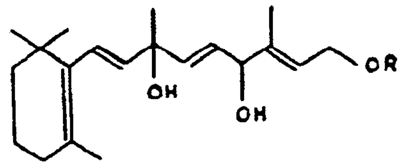

(VII)

avec R =

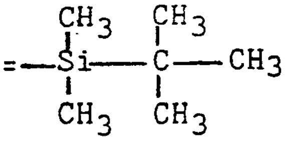

A 1,20 g du triol de formule (VIa) dans 50 ml de diméthylformamide, on ajoute un équivalent de t-butyldiméthylsilane (0,57 g) et 2 équivalents d'imidazole (0,51 g). La solution est agitée pendant 6 heures à température ambiante et diluée par 500 ml d'eau. Le produit est extrait à l'éther (3 × 40 ml). Les phases éthérées sont lavées par une solution saturée de $NH_4Cl$ (3 × 40 ml). Après séchage sur $SO_4Mg$ et évaporation du solvant, on obtient 1,65 g (rendement quantitatif) d'une huile jaune utilisable sans autre purification dans l'étape suivante.

Le rendement en produit (VII) isolé est d'environ 80% et l'analyse par résonance magnétique nucléaire (200 MHz, $CDCl_3$) a donné un spectre superposable à celui du triol de départ avec deux pics supplémentaires: δ : 0,11 (s, 6H, $2CH_3$) ; 0,90 (s, 9H, t.Bu).

e) Préparation de l'éther tout-trans de la vitamine A de formule (VIII)

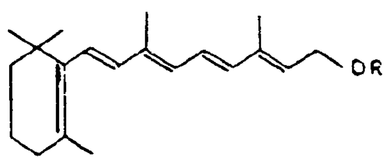

(VIII)

avec R = t-butyldiméthylsilane.

La préparation du titane Ti° décrite par Walborsky (H.M. Walborsky, H.M. Wüst, J. Am. Chem. Soc. 1982, 104, 5807) a été modifiée par l'utilisation de $LiAlH_4$ dosé. Deux équivalents de $TiCl_3$ (1,43 g) sont pesés dans un ballon préalablement séché à l'étuve. On ajoute 30 ml de tétrahydrofuranne anhydre et un équivalent de $LiAlH_4$ dosé dans l'éther (0,176 g) sous argon.

Le mélange est agité pendant 10 minutes à température ambiante et on ajoute alors 0,8 équivalent (1,61 g) du diol de formule (VII) obtenu à l'étape précédente en solution dans 20 ml de tétrahydrofuranne. Après avoir laissé pendant une heure à température ambiante, le milieu est hydrolysé par 10 ml d'eau et 60 ml d'HCl décinormal.

Les phases sont séparées et la phase aqueuse extraite à l'éther (2 × 30 ml). Les phases organiques sont ensuite lavées par 60 ml d'une solution saturée de NaCl, puis séchées sur $SO_4Mg$ et évaporées.

On obtient ainsi 1,30 g d'une huile jaune, soit un rendement de 85% environ, utilisable directement. L'analyse par résonance magnétique nucléaire (200 MHz, $CDCl_3$) a donné un spectre superposable à celui de la vitamine A commerciale avec 2 pics supplémentaires :

δ : 0,11 (s, 6H, $2CH_3$) 0,90 (s, 9H, T. Bu)

Exemple 2

Préparation de l'éther 13-cis de la vitamine A de formule (IX')

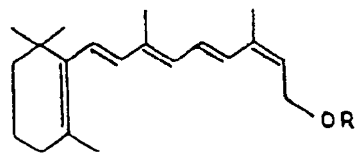

(IX')

avec R = 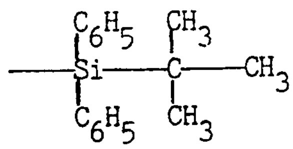 (t-butyldiphénylsilane)

a) Préparation du composé de formule (V') :

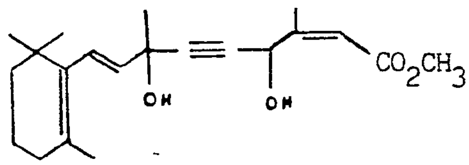

(V')

On dissout 1 g de buténolide [préparé selon C.G. WERMUTH, J. Org. Chem. 1981, (46), 4889] dans 25 $cm^3$ de tétrahydrofuranne anhydre. On ajoute prudemment 0,210 g de NaH préparé à partir d'hydrure de sodium commercial par agitation vigoureuse dans l'hexane anhydre sous argon pendant 30 minutes puis filtration de la suspension ainsi obtenue, double rinçage au pentane anhydre et séchage prolongé sous vide. Le mélange réactionnel sous argon est ensuite refroidi à −78°C et agité pendant une heure. On ajoute 1,7 g de fluoroborate de triméthyloxonium et on laisse remonter la température lentement. Le mélange réactionnel est versé sur 50 $cm^3$ de solution saturée de chlorure d'ammonium. La phase aqueuse est extraite par 25 $cm^3$ d'éther. Les phases organiques sont combinées, lavées avec une solution saturée de chlorure de sodium (2 x 50 $cm^3$), séchées sur sulfate de sodium et évaporées. On obtient ainsi 1,2 g d'un liquide incolore pur correspondant au composé de formule (IV'), soit un rendement de l'ordre de 95%. Le schéma réactionnel a été le suivant :

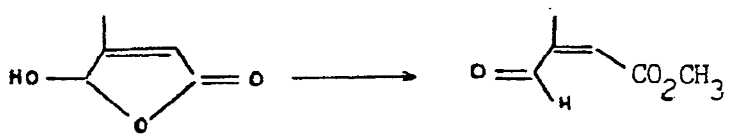

(IV')

On reprend ensuite le mode opératoire de l'étape b) de l'exemple 1 en remplaçant le composé de formule (IV) par le composé de formule (IV') obtenu comme il vient d'être indiqué. L'avancement de la réaction peut être suivi par chromatographie sur couche mince de silice (éluant : acétate d'éthyle/n-hexane dans le rapport volumique 30/70 ; Rf = 0,25). Le rendement de cette réaction en composé (V') est d'environ 100%.

L'analyse par résonance magnétique nucléaire (200 MHz, $CDCl_3$) a donné les résultats suivants :
δ : 0,98 (s, 6H, $CH_3$ en 1) ; 1,42-1,66 (m, 4H, $CH_2$ en 2 et 3) ; 1,66 (s, 3H, $CH_3$ en 9) ; 1,98 (t mal résolu, 2H, $CH_2$ en 4, J = 6Hz) ; 2,10 (s, 3H, $CH_3$ en 5) ; 2,24 (s, 3H, $CH_3$ en 13) ; 4,89 (s, 1H, $H_{12}$) ; 5,55 (d, 1H, $H_8$, J = 16 Hz) ; 6,00 et 6,12 (2s, $H_{14}$ correspondant aux 2 diastéréoisomères $C_{12}$-$C_9$) ; 6,35 (d, 1H, $H_7$, J = 16 Hz).

b) Préparation du composé de formule VI')

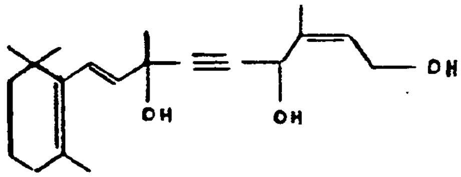

(VI')

On dissout 6 g de composé (V') dans 250 $cm^3$ de toluène anhydre, sous argon. On ajoute, à 0°C, 50 $cm^3$ d'une solution 1 M d'hydrure de diisobutylaluminium.

L'avancement de la réaction peut être suivie par chromatographie sur couche mince de silice (éluant : acétate d'éthyle/n-hexane dans le rapport volumique 70/30 ; Rf = 0,30) ; après 15 minutes, la réaction est achevée.

On ajoute alors 20 $cm^3$ de méthanol, puis une solution aqueuse d'HCl à 5% en poids jusqu'à pH acide. La phase organique est séparée et la phase aqueuse est extraite par deux fois 150 $cm^3$ d'éther. Les phases organiques sont lavées par 100 $cm^3$ d'eau, puis par 2 fois 100 $cm^3$ de solution saturée de chlorure de sodium.

Après séchage sur sulfate de magnésium et évaporation du solvant, on obtient une huile qui sera utilisée sans autre purification dans l'étape suivante et qui correspond au composé de formule (VI').

c) Préparation du composé de formule (VII') :

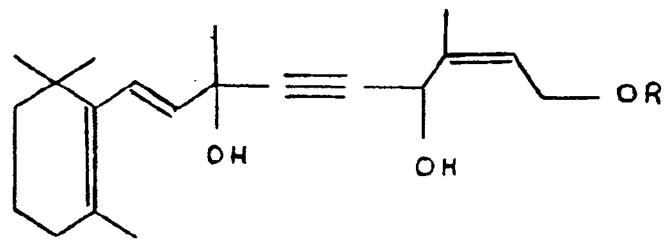

(VII')

avec R = t-butyldiphénylsilane.

Le mode opératoire est identique à celui décrit à l'étape d) de l'exemple 1 mais en utilisant comme matière de départ le composé de formule (VI') obtenu dans l'étape précédente et en remplaçant le chlorure de t-butyldiméthylsilane par le chlorure de t-butyldiphénylsilane. Le composé de formule (VII') obtenu est purifié par chromatographie sur gel de silice (éluant : acétate d'éthyle/n-hexane dans le rapport volumique 20/80).

Le rendement en composé (VII') est de l'ordre de 85% et l'analyse par résonance magnétique nucléaire (200 MHz, $CDCl_3$) a donné les résultats suivants :
δ : 0,99 (s, 6H $CH_3$ en 1) ; 1,05 (s, 9H, t-Bu) ; 1,42-1,64 (m, 4H, $CH_2$ en 2 et 3) ; 1,59, 1,61, 1,67 (3 s, 3 × 3 H, $CH_3$ en 5,9 et 13) ; 1,98 (t, 2H, $CH_2$ en 4, J = 6Hz) ; 4,28 (d, 2H, $CH_2$ en 15, J = 6 Hz) ; 4,79 (d, 1H, $H_{12}$, J = 5 Hz) ; 5,56 (d, 1H, $H_8$ ; J = 16 Hz) ; 5,82 (t, 1H, $H_{14}$, J = 6Hz) ; 6,37 (d, 1H, H7, J = 16 Hz) ; 7,36-7,45 et 7,66-7,71 (10 H, H aromatiques).

d) Préparation des composés de formule (VIII'a) et (VIII'b) :

A partir du composé de formule (VII') obtenu ci-dessus, on peut obtenir soit l'isomère 10-trans de formule (VIII'a), soit l'isomère 10-cis de formule (VIII'b).

1) Isomère 10-trans de formule (VIII'a) :

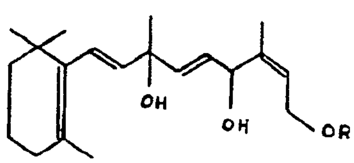

(VIII'a)

avec R = t-butyldiphénylsilane.

On dissout 2 g de composé de formule (VII') dans 100 $cm^3$ d'éther anhydre sous argon à 0°C. On ajoute 1,2 équivalent d'une solution titrée de $LiAlH_4$ dans l'éther. Après avoir laissé pendant une heure à 0°C, on verse le mélange sur 80 $cm^3$ d'une solution saturée de chlorure d'ammonium, puis on dilue par 50 $cm^3$ d'eau. Après séparation de la phase organique, la phase aqueuse est extraite deux fois par 50 $cm^3$ d'éther. Les phases organiques sont réunies et lavées par 100 $cm^3$ d'une solution saturée de chlorure de sodium, puis séchées sur sulfate de magnésium et évaporées. On obtient une huile qui est purifiée par chromatographie sur gel de silice (éluant acétate d'éthyle/n-hexane dans le rapport volumique 30/70).

Le rendement de cette réaction est de l'ordre de 65%, et l'analyse par résonance magnétique nucléaire

(200 MHz, $CDCl_3$ a donné les résultats suivants :
δ : 0,97 (s, 6H, $CH_3$ en 1) ; 1,04 (s, 9H, t-Bu) ; 1,41, 1,64 (2s, 6H, $CH_3$ en 5 et 13) ; 1,42 (s, 3H, $CH_3$ en 9) ; 1,45-1,61 (m, 4H, $CH_2$ en 2 et 3) ; 1,96 (t, 2H, $CH_2$ en 4, J = 6 Hz) ; 4,27 (d, 2H, $CH_2$ en 15, J = 6Hz) ; 4,50 (d, 1H, $H_{12}$, J = 6Hz) ; 5,52 (d, 1H, $H_8$, J = 16 Hz) ; 5,68 (t, 1H, $H_{14}$, J = 6 Hz) ; 5,77 (AB, 2H, $H_{10}$ et $H_{11}$, $J_{AB}$ = 16 Hz, Δν = 40 Hz, $H_{11}$ dédoublé par J = 6 Hz) ; 6,07 (d, 1H, $H_7$, J = 16 Hz) ; 7,34-7,43 et 7,66-7,71 (m, 10 H aromatiques).

2) Isomère 10-cis de formule (VIII'b) :

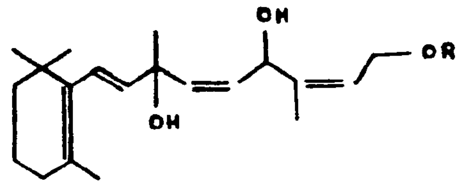

(VIII'b)

où R est le radical t-butyldiphénylsilane.

100 mg de composé (VII') sont dissous dans 5 ml de méthanol absolu. Après addition de 200 mg de catalyseur de LINDLAR (Pd/$CO_3$ Ca désactivé au Pb), l'air contenu dans le réacteur est éliminé sous vide et remplacé par de l'hydrogène. La suspension est alors agitée vigoureusement pendant 72 heures et la réaction suivie en chromatographie sur couche mince (éluant : acétate d'éthyle/n-hexane dans le rapport volumique (80/20 : Rf produit de départ = 0,21 ; Rf des produits diastéréoisomères = 0,26 et 0,32). Le catalyseur est filtré et le solvant évaporé. Les deux diastéréoisomères ont été séparés par chromatographie pour identification.

Le rendement de cette réaction est d'environ 80% et l'analyse par résonance magnétique nucléaire (200 MHz, (200 MHz, $CDCl_3$) a donné les résultats suivants :
Diastéréoisomère Rf = 0,26 : δ : 0,99 (s, 6H, $CH_3$ en 1) ; 1,05 (s, 9H, t-Bu), 1,48 (s, 6H) et 1,67 (s, 3H) ($CH_3$ en 5, 9 et 13) ; 1,43-1,65 (m, 4H, $CH_2$ en 2 et 3) ; 1,98 (t, 2H, $CH_2$ en 4, J = 6 Hz) ; 2,93 (m, 2H, OH) ; 4,27 (d, 2H, $CH_2$ en 15, J = 6 Hz) ; 5,06 (d, 1H, $H_{12}$, J = 7Hz) ; 5,58 (AB, 2H, $H_{10}$ et $H_{11}$, J = 12 Hz, Δν = 46,5 Hz, $H_{11}$ dédoublé par J = 7Hz) ; 5,70 (t, 1H, $H_{14}$, 6 Hz) ; 5,87 (AB, 2H, $H_7$ et $H_8$, J = 16 Hz, Δν = 105 Hz) ; 7,34-7,44 et 7,67-7,72 (m, 10 H aromatiques).
Diastéréoisomère Rf = 0,32 : δ : 0,99 (s, 6H, $CH_3$ en 1) ; 1,05 (s, 9H, t-Bu) ; 1,42-1,67 (m, 4H, $CH_2$ en 2 et en 3) ; 1,47 (s, 3H, $CH_3$ en 9) ; 1.49 (s, 3H, $CH_3$ en 5) ; 1,66 (s, 3H, $CH_3$ en 13) ; 1,98 (t, 2H, $CH_2$ en 4, J = 6 Hz); 2,8 (m, 2H, OH) ; 4,28 (d, 2H, $CH_2$ en 15, J = 6 Hz) ; 5,11 (d, 1H, $H_{12}$, J = 6 Hz) ; 5,54 (AB, 2H, $H_{10}$ et $H_{11}$, J = 12,5 Hz, Δν = 48,5 Hz, $H_{11}$ dédoublé par J = 6 Hz) ; 5,61 (d, 1H, $H_8$, J = 16 Hz) ; 5,71 (t, 1H, $H_{14}$, J = 6 Hz) ; 6,10 (d, 1H, $H_7$, J = 16 Hz) ; 7,34-7,47 et 7,64-7,72 (m, 10 H aromatiques).

e) Préparation de l'éther 13-cis de la vitamine A de formule (IX')

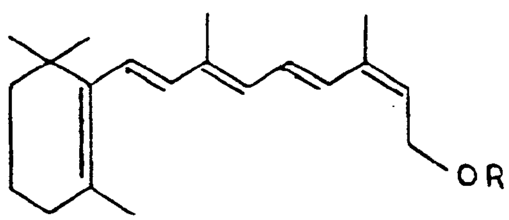

(IX')

où R est le radical t-butyldiphénylsilane.

On utilise le mode opératoire décrit à l'étape e) de l'exemple 1. Le composé de formule (VII) est remplacé par le composé de formule (VIII'a) ou (VIII'b) (10-cis ou 10-trans). Le rendement est de l'ordre de 100%.

L'analyse par résonance magnétique nucléaire (200 MHz, $CDCl_3$) a donné les résultats suivants :
δ : 1,04 (s, 6H, $CH_3$ en 1) ; 1,06 (s, 9H, t-butyl) ; 1,43-1,64 (m, 4H, $CH_2$ en 2 et 3) ; 1,65, 1,73, 1,96 (3s, 3 × 3H, $CH_3$ en 5,9, 13) ; 2,03 (t, 2H, $CH_2$ en 4, J = 6 Hz) ; 4,38 (d, 2H, $CH_2$ en 15, J = 6 Hz) ; 5,71 (t, 1H, $H_{14}$, J = 6 Hz) ; 6,11 (d, 1H, $H_{10}$, J = 11 Hz) ; 6,14 (s, 2H, $H_7$ et $H_8$) ; 6,29 (d, 1H, $H_{12}$, J = 15 Hz) ; 6,55 (dd, 1H, $H_{11}$, J = 15 et 11 Hz) ; 7,34-7,45 (m, 6H aromatiques) ; 7,65-7,74 (m, 4H aromatiques).

Dans les deux exemples ci-dessus, l'étape déterminante est la réduction stéréospécifique par un mélange

$LiAlH_4/TiCl_3$ des composés (VII), (VIII'a) ou (VIII'b) en éthers de la vitamine A respectivement tout-trans de formule (VIII) ou 13-cis de formule (IX'). Cette réduction se fait avec un rendement de 100%.

On remarquera que l'on utilise, dans ces deux exemples, des solutions titrées d'hydrure de lithium et d'aluminium et que les réactions des étapes e) des exemples 1 et 2 sont effectuées à température ambiante et non au reflux.

Exemple 3

Préparation de l'éther 13-cis de la vitamine A de formule (IX")

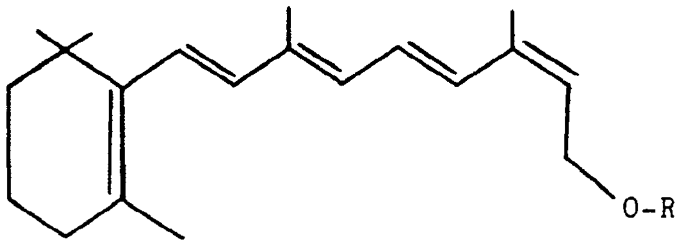

(IX")

où R représente le radical triéthylsilane.

a) Préparation du composé de formule (VIb) :

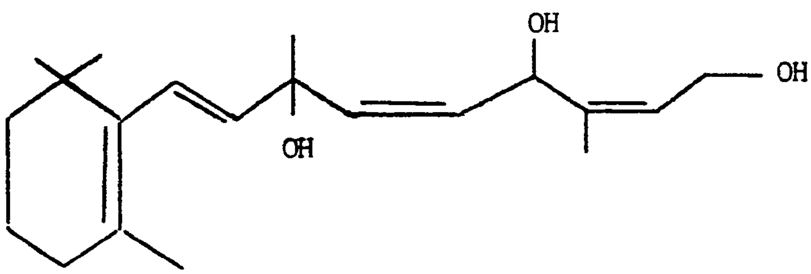

(VIb)

On dissout 1 g de composé (VI') obtenu à la fin de l'étape b) de l'exemple 2 dans 10 cm³ de méthanol. On ajoute une quantité catalytique du catalyseur de LINDLAR. On agite à température ambiante pendant 16 heures sous atmosphère d'hydrogène à la pression atmosphérique. Après filtration du catalyseur et distillation du solvant sous pression réduite, on récupère 1 g d'huile incolore (composé de formule (VIb)), qui est utilisée sans autre purification dans la préparation suivante.

b) Préparation du composé de formule (VIII'b)

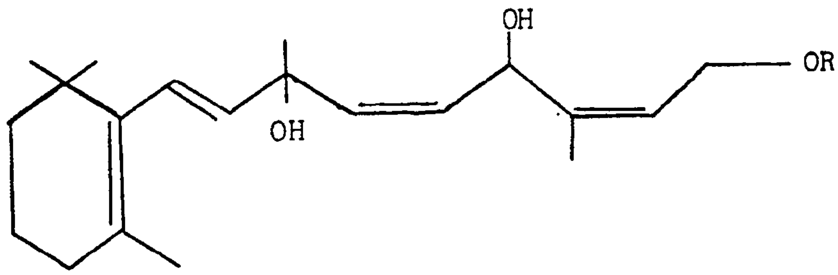

(VIII'b)

avec R = 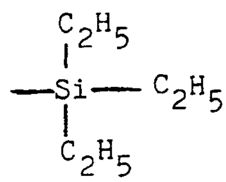

Le mode opératoire est analogue à celui de l'étape d) de l'exemple 1, le chlorodiméthyltertiobutylsilane étant néanmoins remplacé par le chlorotriéthylsilane. Le rendement de cette réaction est de 90%. Le composé (VIII'b) est utilisé sans autre purification dans l'étape suivante.

c) Préparation du composé de formule (IX") :

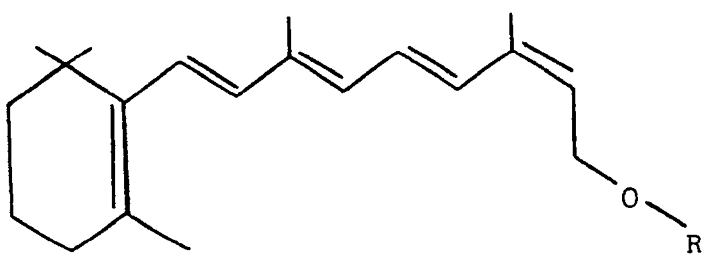

(IX")

où R a la même signification qu'à l'étape précédente.

Le mode opératoire est identique à celui de l'étape e) de l'exemple 1.

La réaction est suivie en chromatographie sur couche mince de silice. Dès que le produit de départ a disparu, le milieu réactionnel est filtré rapidement sur gel de silice et le solvant est distillé sous pression réduite. Le composé (IX") est obtenu avec un rendement quantitatif. Il est utilisé sans autre purification dans l'étape suivante.

Exemple 4

Préparation du 13-cis rétinol de formule (X") :

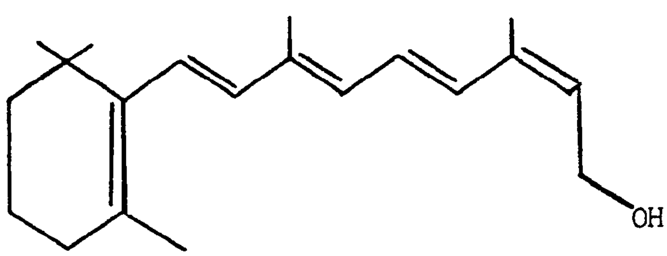

(X")

1 g de composé de formule (IX″) obtenu à l'exemple 3 est dissous dans 15 $cm^3$ d'un mélange (tétrahydrofuranne-acétonitrile) contenant 1/3 de tétrahydrofuranne. La solution ainsi obtenue est traitée par 5 équivalents de fluorure de sodium anhydre et 0,1 équivalent de fluorure de pyridinium pur parfaitement sec et broyé en fines particules juste avant l'emploi. La réaction est faite à l'abri de la lumière, sous argon, après avoir ajouté à la solution 2 g d'un agent déshydratant constitué par un "tamis moléculaire" de 4 Å.

Après 5 heures d'agitation à température ambiante, le mélange réactionnel est traité par 10 $cm^3$ d'une solution saturée en chlorure d'ammonium et 10 $cm^3$ d'eau. Après décantation, la phase aqueuse est extraite par deux fois 10 $cm^3$ d'éther. Les phases organiques sont combinées et lavées par 20 $cm^3$ d'une solution saturée en chlorure de sodium. Après séchage sur sulfate de sodium, le solvant est distillé à température ambiante sous pression réduite.

Le rendement de cette réaction est supérieur à 80%. On obtient 1 g d'une huile jaune vif donnant une seule tâche en chromatographie sur couche mince de gel de silice (éluant : éther/hexane dans le rapport volumique 10/90 ; Rf = 0,10).

L'analyse du produit obtenu par résonance magnétique nucléaire (200 MHz, $CDCl_3$) a donné un spectre identique à celui décrit par R.S.H. LIU et A.E. ASATO, Tetrahedron, 40 (11), 1931 (1984), pour le 13-cis-rétinol.

Exemple 5

Préparation du 13-cis rétinal de formule (XI″) :

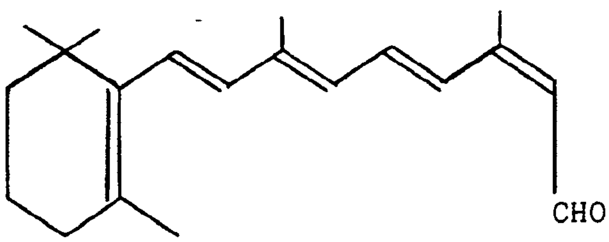

(XI")

On dissout 1 g de 13-cis- rétinol obtenu à l'exemple 4 dans 15 $cm^3$ de tétrachlorure de carbone anhydre à l'abri de la lumière. On ajoute, sous agitation vigoureuse, 1,5 g (5 équivalents) de $MnO_2$ fraîchement préparé. Après 30 minutes à température ambiante, la suspension est filtrée rapidement sur gel de silice et le solvant est distillé à température ambiante sous pression réduite.

On obtient ainsi 1 g de 13-cis-rétinal donnant une seule tâche en chromatographie sur couche mince de gel de silice (éluant : éther/hexane dans le rapport volumique 10/90 ; Rf = 0,25).

L'analyse par résonance magnétique nucléaire (200 MHz, $CDCl_3$) a donné un spectre identique à celui décrit par R.S.H. LIU et A.E. ASATO, Tetrahedron, 40 (II), 1931 (1984) pour le 13-cis-rétinal.

Exemple 6

Préparation de l'acide 13-cis-rétinoïque de formule (XII″) :

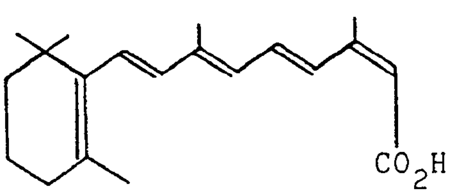

(XII")

a) Préparation de l'ester éthylique de l'acide 13-cis-rétinoïque

On dissout 1 g de 13-cis-rétinal brut obtenu à l'exemple 5 dans15 $cm^3$ d'éthanol. On ajoute successivement 5 équivalents de cyanure de sodium (0,86 g) et 6 équivalents de AgO (2,6 g). La suspension ainsi obtenue est agitée à 40°C à l'abri de la lumière pendant 14 heures. Le milieu réactionnel est filtré et la solution est chromatographiée sur gel de silice (éluant : acétate d'éthyle/hexane dans le rapport volumique 2/98). On obtient

ainsi 0,65 g d'ester éthylique de l'acide 13-cis-rétinoïque ; le rendement est de 60% environ.

L'analyse par résonance magnétique nucléaire (200 MHz, $CDCl_3$) a donné un specte identique à celui décrit par R.S.H. LIU et A.E. ASATO, Tetrahedron, 40 (II), 1931 (1984) pour l'ester méthylique de l'acide 13-cis rétinoïque à l'exception du signal caractéristique du groupement méthyle.

b) Hydrolyse

On met en suspension 0,100 g de l'ester obtenu ci-dessus dans 5 $cm^3$ d'éthanol et 5 $cm^3$ de solution aqueuse 6M de soude. On agite à 50°C à l'abri de la lumière jusqu'à ce que tout le solide soit dissous.

Le mélange réactionnel est alors dilué par 100 $cm^3$ d'eau et l'éthanol est distillé sous pression réduite. La phase aqueuse est acidifiée jusqu'à pH 2 par addition d'acide chlorhydrique en solution aqueuse à 10%. On extrait par trois fois 10 $cm^3$ d'éther. On sèche sur sulfate de sodium et on distille le solvant sous pression réduite. On récupère un solide jaune vif qui est recristallisé dans le méthanol. On obtient ainsi l'acide 13-cis rétinoïque avec un rendement de 95%.

L'analyse par résonance magnétique nucléaire (200 MHz, $CDCl_3$) a donné un spectre identique à celui décrit par R.S.H. LIU et A.E. ASATO, Tetrahedron, 40 (II), 1931 (1984) pour l'ester méthylique de l'acide 13-cis rétinoïque, à l'exception du signal caractéristique du reste méthyle.

On a analysé cet acide 13-cis rétinoïque par chromatographie en utilisant une colonne Lichrosorb RP 18 (5 μ), commercialisée par la Société MERCK, dont la hauteur est de 25 cm et le diamètre de 0,4 cm. L'éluant est un mélange constitué de 25% en volume d'une solution aqueuse à 0,2% en volume d'acide acétique et de 75% en volume d'une solution d'acide acétique à 0,2% en volume dans du méthanol. Le débit est réglé à 1 ml/mn. Le temps de rétention est de 19 mn.

Le chromatogramme obtenu est superposable à celui d'un échantillon d'acide 13-cis rétinoïque commercial.

Exemple 7

Préparation de la vitamine A tout-trans de formule (IX)

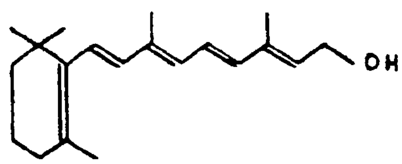

(IX)

A partir de l'éther tout-trans de la vitamine A de formule (VIII) obtenu à l'issue de l'étape e) de l'exemple 1, on peut obtenir la vitamine A tout-trans, stéréo-spécifiquement pure.

A 1 équivalent d'éther silylé de vitamine A précédemment obtenu (1,25 g) en solution dans 20 ml de tétrahydrofuranne, on ajoute 1,5 équivalent de fluorure de tétrabutylammonium. Le mélange est agité pendant 2 heures à température ambiante.

Le mélange réactionnel est ensuite filtré sur silice, puis la silice est lavée à l'éther (2 × 20 ml). Après avoir évaporé le solvant, le produit est chromatographié (éluant : acétate d'éthyle/n-hexane dans le rapport volumique 20/80 ; Rf = 0,20).

On obtient ainsi 740 mg de vitamine A sous forme d'un solide jaune, soit un rendement d'environ 80%, dont l'analyse par résonance magnétique nucléaire (200 MHz, $CDCl_3$) a donné un spectre superposable à celui de la vitamine A commerciale :

δ : 1,02 (s, 6H, $CH_3$ en 1) ; 1,36-1,68 (m, 4H, $CH_2$ en 2 et en 3) ; 1,71 (s, 3H, $CH_3$ en 5) ; 1,87 (s, 3H, $CH_3$ en 9) ; 2,02 (t, 2H, J = 6 Hz, $CH_2$ en 4) ; 4,31 (d, 2H, J = 7 Hz, $CH_2$ en 15) ; 5,69 (t, 1H, $H_{14}$, J = 7 Hz) ; 6,10 (d, 1H, $H_{10}$, J = 11 Hz) ; 6,13 et 6,14 (2s, 2H, $H_7$ et $H_8$) ; 6,28 (d, 1H, $H_{12}$, J = 16 Hz) ; 6,62 (dd, 1H, J = 11 Hz et J = 16 Hz, $H_{11}$).

Exemple 8

Préparation du rétinal tout-trans de formule (X)

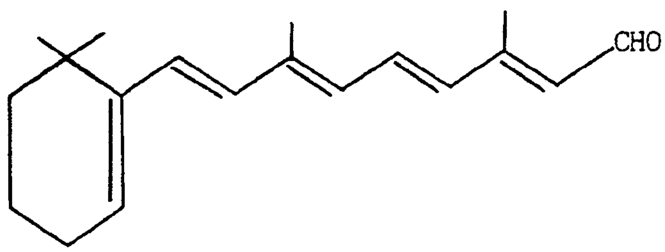

(X)

A 1 gramme de vitamine A tout-trans de formule (IX) obtenue à l'exemple 7, dissous dans 15 ml de $CH_2Cl_2$,on ajoute 1,5 g (5 équivalents) de $MnO_2$ préparé selon le procédé décrit par J. Attenburrov, A.F.B. Cameron, J.K. Chapman, R.M. Evans, B.A. Hens, A.B.A. Janssen, et T. Walker dans J. Chem. Soc. 1952, 1094.

La suspension est agitée à l'abri de la lumière pendant 2 heures. La réaction est quantitative. Les composés minéraux sont enlevés par filtration. Après évaporation du solvant, il reste 1 gramme d'un solide jaune vif pur de formule (X).

L'analyse par résonance magnétique nucléaire (200 MHz, $CDCl_3$) a donné les résultats suivants :
δ : 1,04 (s, 6H, $CH_3$ en 1) ; 1,45-1,70 (m, 4H, $CH_2$ en 2 et 3) ; 1,73 (s, 3H, $CH_3$ en 5) ; 2,00-2,03 (signal large, 5H, $CH_2$ en 4 et $CH_3$ en 9) ; 2,33 (s, 3H, $CH_3$ en 13) ; 5,98 (d, 1H, $H_{14}$, J = 8,0 Hz) ; 6,20 (d, 1H, $H_{10}$, J = 12,0 Hz) ; 6,27 (AB, 2H, $H_7$ et $H_8$), J = 16,5 Hz, $\Delta v$ = 36 Hz) ; 6,37 (d, 1H, $H_{12}$, J = 15,4 Hz) ; 7,15 (dd, 1H, $H_{11}$, J = 15,4 et 12,0 Hz) ; 10,12 (d, 1H, $H_{15}$, J = 8,0 Hz).

Exemple 9

Préparation de l'acide rétinoïque tout-trans de formule (XI)

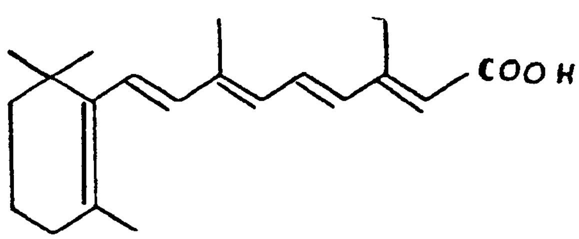

(XI)

a) On prépare tout d'abord de l'oxyde d'argent AgO par le procédé de F. JIRSA (Z. Anorg. Allgem. Chem. 1935, 225, 302), en ajoutant à 7 g de nitrate d'argent dissous dans 25 g d'eau une solution de 15 g (2 équivalents) de KMnO4 dans 300 ml d'eau, puis 15 g (6 équivalents) de KOH dissous dans 75 ml d'eau. La suspension résultante est agitée pendant 2 heures à température ambiante puis filtrée. Le résidu est lavé à l'eau jusqu'à ce que les eaux de lavage soient incolores. On obtient ainsi un solide noir, qui est mis à l'étuve à 40°C pendant 2 heures. La composition du produit ainsi obtenu est d'environ 60% AgO et 40% $Ag_2O$.

b) On procède ensuite à une oxydation du rétinal selon le procédé de préparation décrit par E.J. Corey, N.W. Gilman, B.E. Garnem [J. Am. Chem. Soc., 90, (1968), 5616].

On dissout 200 mg du rétinal de l'exemple 8 dans 20 ml de méthanol contenant 0,05% en poids d'eau et on ajoute 1,4 g (15 équivalents) d'oxyde d'argent AgO précédemment préparé et 180 mg (5 équivalents) de NaCN. La suspension est maintenue agitée pendant 18 heures à l'abri de la lumière.

Les sels insolubles sont alors éliminés par filtration sur silice et on soumet le produit à une chromatographie (éluant : acétate d'éthyle/n-hexane dans le rapport volumique 30/70 ; Rf du rétinal = 0,70 ; Rf de l'acide rétinoïque = 0,25).

Le rendement en acide rétinoïque tout-trans est d'environ 60% et l'analyse par résonance magnétique nucléaire (200 MHz, $CDCl_3$) a donné les résultats suivants :
δ : 1,03 (s, 6H, $CH_3$ en 1) ; 1,44-1,62 (m, 4H, $CH_2$ en 2 et 3) ; 1,72 (s, 3H, $CH_2$ en 5) ; 2,01-2,06 (pic large, 5H,

$CH_3$ en 9 et $CH_2$ en 4) ; 2,37 (s, 3H, $CH_3$ en 13) ; 5,80 (s, 1H, $H_{14}$) ; 6,14 (d, 1H, J = 16 Hz, $H_8$) ; 6,15 (d, 1H, $H_{10}$, J = 11 Hz) ; 6,31 (d, 1H, $H_{12}$, J = 15 Hz) ; 7,05 (dd, 1H, $H_{11}$, J = 11 et 15 Hz).

Ce spectre est superposable à celui de l'acide rétinoïque tout-trans commercial.

On a aussi analysé cet acide rétinoïque tout-trans par chromatographie en utilisant une colonne Lichrosorb RP 18 (5 µ), commercialisée par la Société MERCK, dont la hauteur est de 25 cm et le diamètre de 0,4 cm. L'éluant est un mélange constitué de 25% en volume d'une solution aqueuse à 0,2% en volume d'acide acétique et 75% en volume d'une solution d'acide acétique à 0,2% en volume dans du méthanol. Le débit est réglé à 1 ml/mn. Le temps de rétention est de 100 minutes environ.

Le chromatogramme obtenu est superposable avec celui d'un échantillon d'acide rétinoïque tout-trans commercial.

## Revendications

1. Procédé de synthèse de composés tout-trans pris dans le groupe constitué par la vitamine A et ses éthers, le rétinal et l'acide rétinoïque, et leurs isomères 13-cis, caractérisé par le fait que l'on réalise une réduction stéréospécifique des deux groupes hydroxyles d'un éther-diol, précurseur de la vitamine A, par un mélange (trichlorure de titane, hydrure d'aluminium et de lithium) en milieu solvant anhydre, à une température comprise entre 5°C et 40°C environ et qu'éventuellement, on transforme l'éther obtenu en vitamine A, rétinal ou acide rétinoïque.

2. Procédé selon la revendication 1, caractérisé par le fait que l'on fait réagir 0,8 équivalent d'un éther-diol, précurseur de la vitamine A, avec 2 équivalents de trichlorure de titane et 1 équivalent d'hydrure d'aluminium et de lithium.

3. Procédé selon l'une des revendications 1 ou 2, caractérisé par le fait que l'on fait réagir l'éther-diol, précurseur de la vitamine A, à une température voisine de 20°C.

4. Procédé selon l'une des revendications 1 à 3, caractérisé par le fait que le solvant anhydre est un éther tel que l'éther diéthylique, le dioxane, le tétrahydrofuranne ou leurs mélanges.

5. Procédé selon l'une des revendications 1 à 4, caractérisé par le fait que l'éther-diol, précurseur de la vitamine A, est choisi dans le groupe constitué par :

— l'éther-diol tout-trans de formule (VII) :

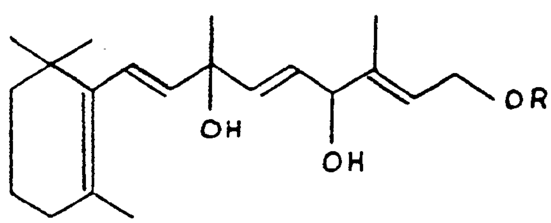

(VII)

formule dans laquelle R est un reste alkyle en $C_1$-$C_6$, un reste aralkyle éventuellement substitué par un ou plusieurs groupements alcoxy, tels que méthoxy-4'-benzyle et diméthoxy-3',4' benzyle, ou un reste

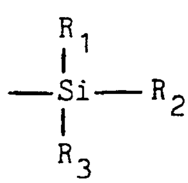

dans lequel $R_1$, $R_2$, $R_3$ identiques ou différents, représentent un reste alkyle en $C_1$-$C_6$, linéaire ou ramifié, ou un reste aryle, éventuellement substitué ;

— l'éther-diol 10-trans de formule (VIII'a) :

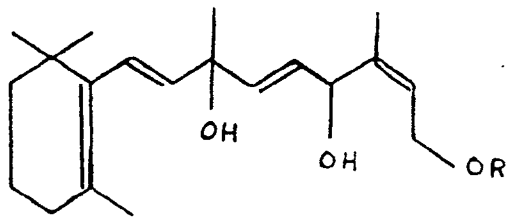

(VIII'a)

et

— l'éther-diol 10-cis de formule (VIII'b) :

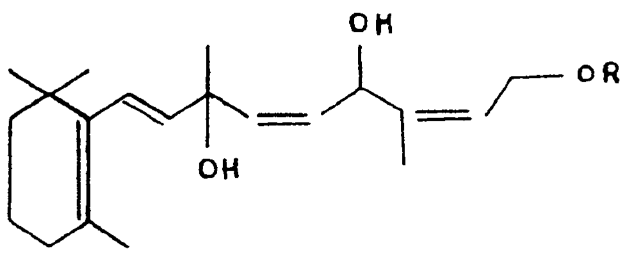

(VIII' b)

formules dans lesquelles R a les significations susmentionnées.

6. Procédé selon la revendication 5, caractérisé par le fait que l'on obtient l'éther-diol tout-trans, précurseur de la vitamine A, de formule (VII) par éthérification du triol correspondant de formule (VIa) :

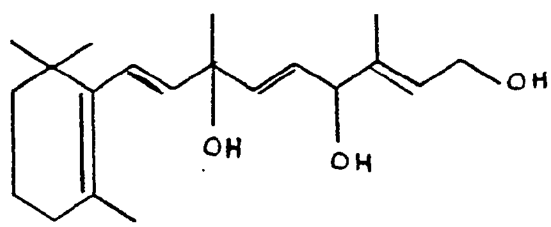

(VIa)

par un reste alkyle en $C_1$-$C_6$, un reste aralkyle éventuellement substitué par un ou plusieurs groupements alcoxy, notamment le méthoxy-4′ benzyle et le diméthoxy-3′, 4′ benzyle, ou un reste

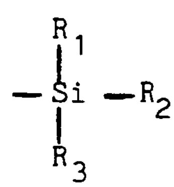

où $R_1$, $R_2$, $R_3$, identiques ou différents, représentent un reste alkyle en $C_1$-$C_6$, ramifié ou linéaire, ou un reste aryle éventuellement substitué, en présence d'imidazole, à température ambiante.

7. Procédé selon la revendication 6, caractérisé par le fait que, pour obtenir le composé de formule (VIa), on fait réagir un ester acétylénique d'éthynyl-β-ionol de formule (V)

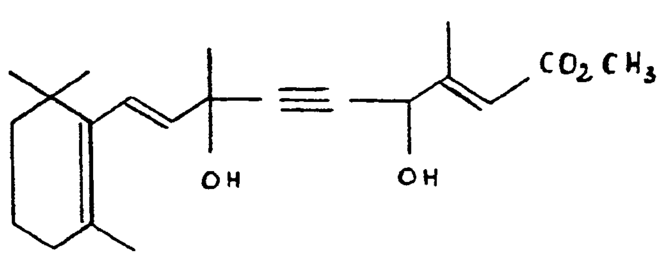

(V)

avec de l'hydrure d'aluminium et de lithium.

8. Procédé selon la revendication 7, caractérisé par le fait que pour obtenir le composé de formule (V), on fait réagir l'éthynyl-β-ionol de formule (III)

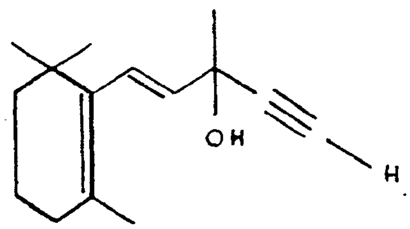

(III)

avec du bromure d'éthylmagnésium puis avec du γ-oxo-sénécioate de méthyle de formule (IV)

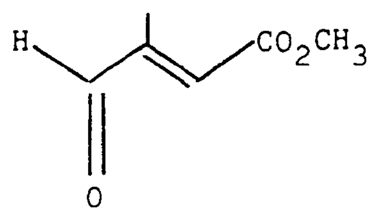

(IV)

9. Procédé selon la revendication 5, caractérisé par le fait que l'on obtient l'éther-diol 10-cis, précurseur de la vitamine A, de formule (VIII'b) par éthérification de la fonction alcool terminal du composé de formule (VIb):

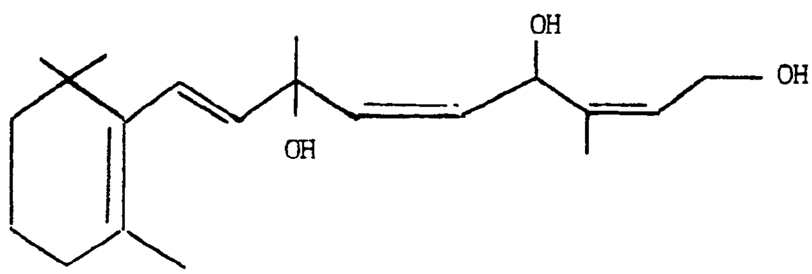

(VIb)

par un reste alkyle $C_1$-$C_6$, un reste aralkyle éventuellement substitué par un ou plusieurs groupements alcoxy, notamment le méthoxy-4′ benzyle et le diméthoxy-3′,4′ benzyle ou d'un reste $SiR_1R_2R_3$ où $R_1$, $R_2$, $R_3$ ont les significations indiquées à la revendication 5, en présence d'imidazole, à température ambiante, suivie d'une extraction à l'éther.

10. Procédé selon la revendication 9, caractérisé par le fait que, pour obtenir le composé de formule (VIb), on réduit le composé de formule (VI')

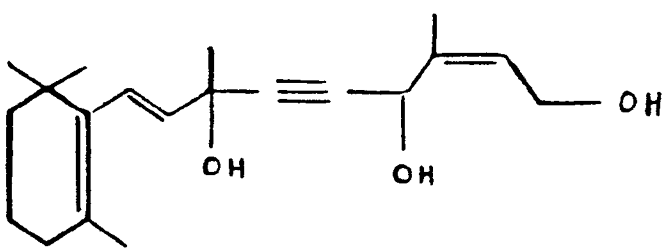

(VI')

par de l'hydrogène, en présence du catalyseur de LINDLAR.

11. Procédé selon la revendication 5, caractérisé par le fait que l'on obtient l'éther-diol 10-cis de formule (VIII'b) par réduction par l'hydrogène, en présence du catalyseur de LINDLAR, du composé de formule (VII')

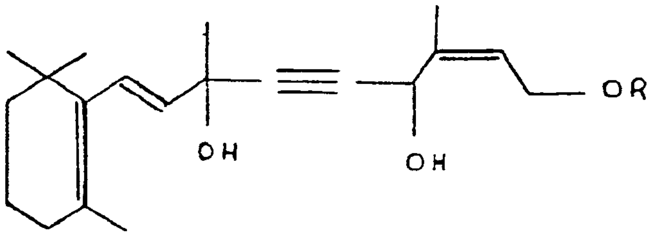

(VII')

formule dans laquelle R a les mêmes significations qu'à la revendication 5.

12. Procédé selon la revendication 5, caractérisé par le fait que l'on obtient l'éther-diol 10-trans de formule (VIII'a) par réduction à l'hydrure d'aluminium et de lithium du composé de formule (VII')

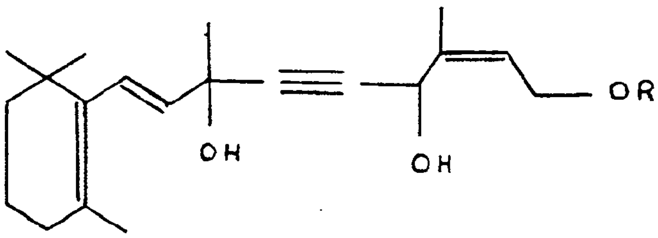

(VII')

formule dans laquelle R a les mêmes significations qu'à la revendication 5, et extraction à l'éther de la phase organique contenant le composé de formule (VIII'a).

13. Procédé selon l'une des revendications 11 ou 12, caractérisé par le fait que l'on obtient le composé de formule (VII') en éthérifiant le composé de formule (VI')

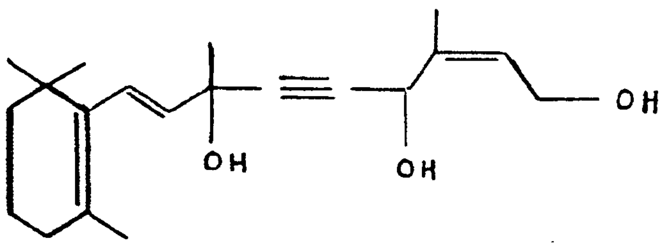

(VI')

un reste alkyle en $C_1$-$C_6$, un reste aralkyle éventuellement substitué par un ou plusieurs groupements alcoxy, notamment le reste de méthoxy-4' benzyle ou le diméthoxy-3', 4' benzyle, ou le reste

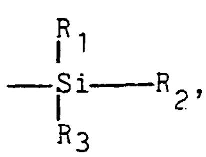

où $R_1$, $R_2$, $R_3$ ont les mêmes significations qu'à la revendication 5, en présence d'imidazole, à température ambiante.

14. Procédé selon la revendication 13, caractérisé par le fait que, pour obtenir le composé de formule (VI'), on fait réagir un composé de formule (V') :

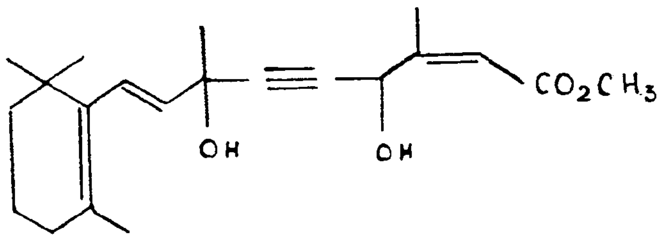

(V')

avec de l'hydrure de diisobutylaluminium.

15. Procédé selon la revendication 14, caractérisé par le fait que, pour obtenir le composé de formule (V'), on fait réagir l'éthynyl-β-ionol de formule (III) avec du bromure d'éthylmagnésium et un composé de formule (IV').

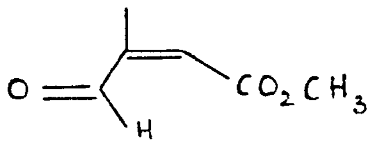

(IV')

16. Procédé selon la revendication 15, caractérisé par le fait que l'on prépare le composé de formule (IV') en faisant réagir un buténolide de formule :

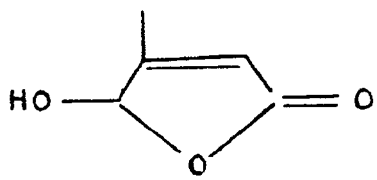

avec de l'hydrure de sodium, puis avec du fluoroborate de triméthyloxonium.

## Patentansprüche

1. Verfahren zur Herstellung von All-trans-Verbindungen, ausgewählt unter Vitamin A und den Ethern davon, Retinal und Retinsäure und deren 13-cis-Isomeren, dadurch gekennzeichnet, daß man eine stereospezifische Reduktion der beiden Hydroxylgruppen eines Etherdiol-Vorläufers von Vitamin A, mit einem Gemisch (Titantrichlorid, Lithiumaluminiumhydrid) in einem wasserfreien Lösungsmittelmilieu bei einer Temperatur im Bereich von etwa 5°C bis 40°C durchführt und gegebenenfalls den erhaltenen Ether in Vitamin A, Retinal oder Retinsäure überführt.

2. Verfahren nach Anspruch 1, dadurch gekennzeichnet, daß man 0,8 Äquivalente eines Etherdiol-Vorläufers von Vitamin A mit 2 Äquivalenten Titantrichlorid und einem Äquivalent Lithiumaluminiumhydrid umsetzt.

3. Verfahren nach einem der Ansprüche 1 oder 2, dadurch gekennzeichnet, daß man den Etherdiol-Vorläufer von Vitamin A bei einer Temperatur im Bereich von 20°C reagieren läßt.

4. Verfahren nach einem der Ansprüche 1 bis 3, dadurch gekennzeichnet, daß das wasserfreie Lösungsmittel ein Ether, wie z.B. Diethylether, Dioxan, Tetrahydrofuran oder eines der Gemische davon, ist.

5. Verfahren nach einem der Ansprüche 1 bis 4, dadurch gekennzeichnet, daß der Etherdiol-Vorläufer von Vitamin A ausgewählt ist unter :

— dem All-trans-Etherdiol der allgemeinen Formel (VII) :

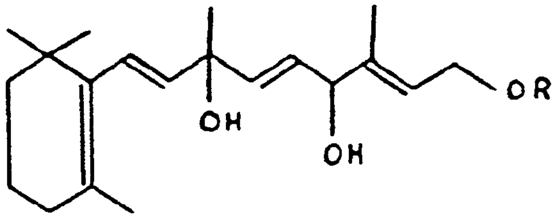

(VII)

worin R einen $C_1$-$C_6$-Alkylrest, einen gegebenenfalls durch eine oder mehrere Alkoxygruppen substituierten Aralkylrest, wie z.B. 4′-Methoxybenzyl oder 3′,4′-Dimethoxybenzyl, oder einen Rest

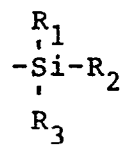

bedeutet, worin $R_1$, $R_2$ und $R_3$ gleich oder verschieden sind und einen geradkettigen oder verzweigten $C_1$-$C_6$-Alkylrest oder einen gegebenenfalls substituierten Arylrest bedeuten ;

— dem 10-trans-Etherdiol der allgemeinen Formel (VIII′a) :

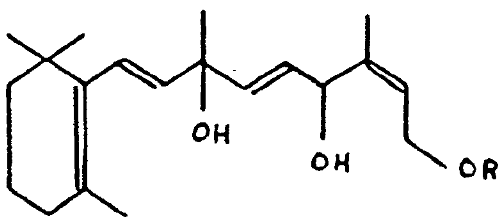

(VIII'a)

und

— dem 10-cis-Etherdiol der allgemeinen Formel (VIII′b) :

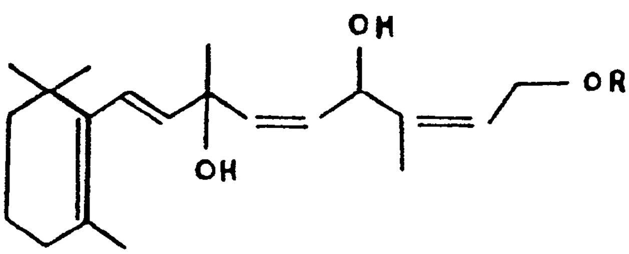

(VIII' b)

worin R die oben angegebenen Bedeutungen besitzt.

6. Verfahren nach Anspruch 5, dadurch gekennzeichnet, daß man den All-trans-Etherdiol-Vorläufer von Vitamin A der allgemeinen Formel (VII) erhält, indem man das entsprechende Triol der Formel (VIa) :

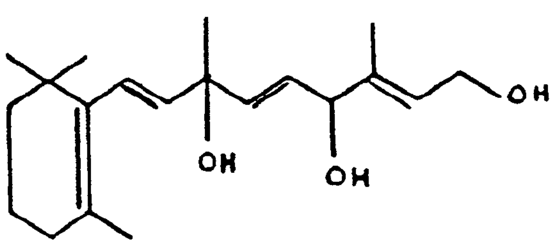

(VI a)

mit einem $C_1$-$C_6$-Alkylrest, mit einem Aralkylrest, der gegebenenfalls durch eine oder mehrere Alkoxygruppen substituiert ist, wie insbesondere dem 4′-Methoxybenzylrest und dem 3′,4′-Dimethoxybenzylrest, oder mit ei-

nem Rest

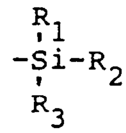

worin $R_1$, $R_2$ und $R_3$ gleich oder verschieden sind und für einen geradkettigen oder verzweigten $C_1$-$C_6$-Alkylrest oder für einen gegebenenfalls substituierten Arylrest stehen, in Gegenwart von Imidazol bei Umgebungstemperatur verethert.

7. Verfahren nach Anspruch 6, dadurch gekennzeichnet, daß man zur Herstellung einer Verbindung der Formel (VIa) einen Acetylenester von Ethinyl-β-ionol der Formel (V) :

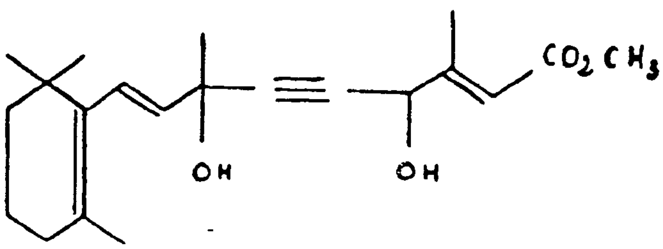

(V)

mit Lithiumaluminiumhydrid umsetzt.

8. Verfahren nach Anspruch 7, dadurch gekennzeichnet, daß man zur Herstellung einer Verbindung der Formel (V) Ethinyl-β-ionol der Formel (III) :

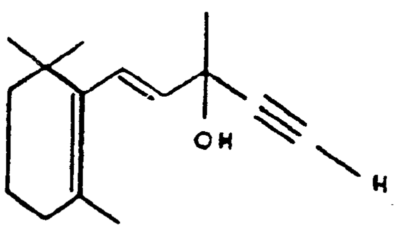

(III)

mit Ethylmagnesiumbromid und anschließend mit γ-Oxomethylsenecioat der Formel (IV) :

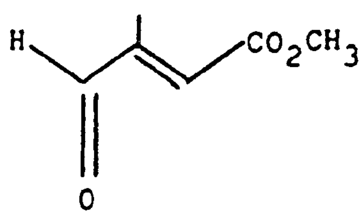

(IV)

umsetzt.

9. Verfahren nach Anspruch 5, dadurch gekennzeichnet, daß man den 10-cis-Etherdiol-Vorläufer von Vitamin A der allgemeinen Formel (VIII'b) erhält, indem man die terminale Alkoholfunktion einer Verbindung der Formel (VIb) :

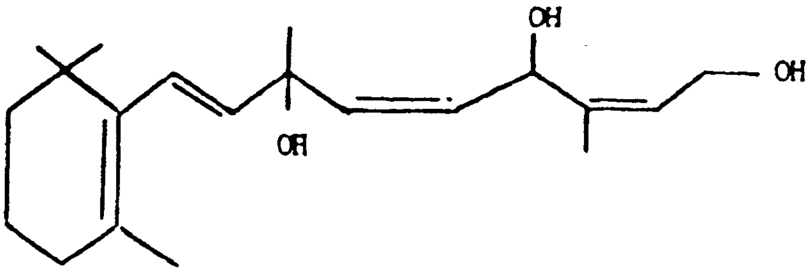

(VIb)

mit einem $C_1$-$C_6$-Alkylrest, mit einem Aralkylrest, der gegebenenfalls durch eine oder mehrere Alkoxygruppen substituiert ist, wie insbesondere dem 4′-Methoxybenzylrest und dem 3′, 4′-Dimethoxybenzylrest, oder mit einem $SiR_1R_2R_3$-Rest, worin $R_1$, $R_2$ und $R_3$ die in Anspruch 5 angegebenen Bedeutungen besitzen, in Gegenwart von Imidazol bei Umgebungstemperatur verethert und eine Etherextraktion durchführt.

10. Verfahren nach Anspruch 9, dadurch gekennzeichnet, daß man zur Herstellung eines Verbindung der Formel (VIb) die Verbindung der Formel (VI′) :

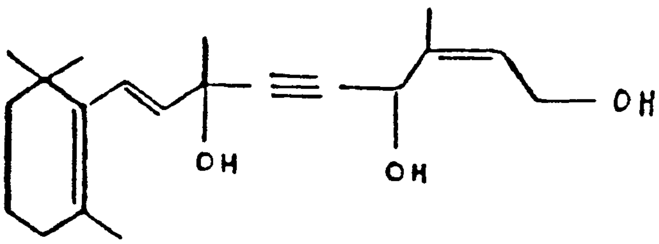

(VI')

mit Wasserstoff in Gegenwart eines LINDLAR-Katalysators reduziert.

11. Verfahren nach Anspruch 5, dadurch gekennzeichnet, daß man das 10-cis-Etherdiol der Formel (VIII′b) durch Reduktion einer Verbindung der allgemeinen Formel (VII′) :

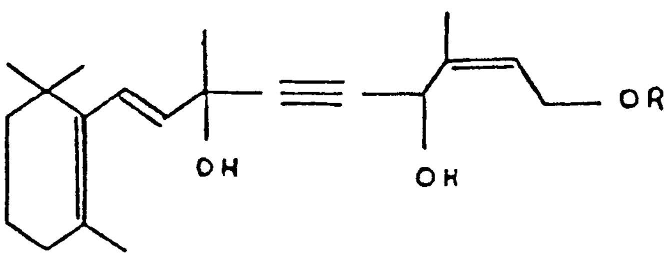

(VII')

worin R die in Anspruch 5 angegebenen Bedeutungen besitzt, mit Wasserstoff in Gegenwart eines LINDLAR-Katalysators reduziert.

12. Verfahren nach Anspruch 5, dadurch gekennzeichnet, daß man das 10-trans-Etherdiol der Formel (VIII′a) durch Reduktion einer Verbindung der allgemeinen Formel (VII′) :

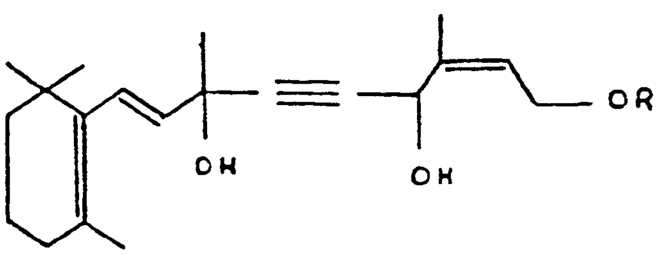

(VII')

worin R die in Anspruch 5 angegebenen Bedeutungen besitzt, mit Lithiumaluminiumhydrid und durch Etherextraktion der die Verbindung der Formel (VIII′a) enthaltenden organischen Phase erhält.

13. Verfahren nach einem der Ansprüche 11 oder 12, dadurch gekennzeichnet, daß man die Verbindung der allgemeinen Formel (VII') erhält, indem man die Verbindung der Formel (VI') :

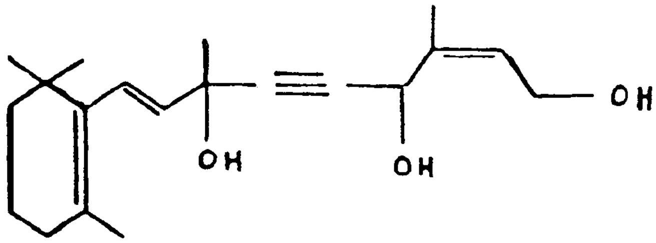
(VI')

mit einem $C_1$-$C_6$-Alkylrest, mit einem Aralkylrest, der gegebenenfalls durch eine oder mehrere Alkoxygruppen substituiert ist, wie insbesondere dem 4'-Methoxybenzylrest und dem 3',4'-Dimethoxybenzylrest, oder mit dem Rest

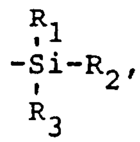

worin $R_1$, $R_2$ und $R_3$ die in Anspruch 5 angegebenen Bedeutungen besitzen, in Gegenwart von Imidazol bei Umgebungstemperatur verethert.

14. Verfahren nach Anspruch 13, dadurch gekennzeichnet, daß man zur Herstellung einer Verbindung der Formel (VI') eine Verbindung der Formel (V') :

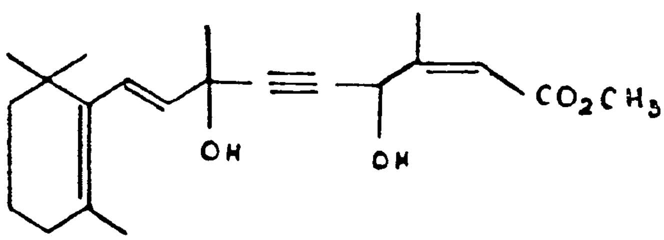
(V')

mit Diisobutylaluminiumhydrid umsetzt.

15. Verfahren nach Anspruch 14, dadurch gekennzeichnet, daß man zur Herstellung der Verbindung der Formel (V') Ethinyl-β-ionol der Formel (III) mit Ethylmagnesiumbromid und einer Verbindung der Formel (IV'):

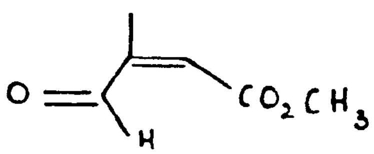
(IV')

umsetzt.

16. Verfahren nach Anspruch 15, dadurch gekennzeichnet, daß man die Verbindung der Formel (IV') herstellt, indem man ein Butenolid der Formel :

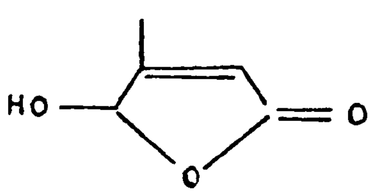

mit Natriumhydrid und anschließend mit Trimethyloxonium-fluorborat umsetzt.

## Claims

1. Process for the synthesis of all-trans compounds taken from the group consisting of vitamin A and its ethers, retinal and retinoic acid and their 13-cis isomers, characterised in that a sterospecific reduction of the two hydroxyl groups of an ether-diol precursor of vitamin A is carried out with a mixture (titanium trichloride, lithium aluminium hydride) in an anhydrous solvent medium at a temperature of approximately between 5°C and 40°C and that, if desired, the ether obtained is converted into vitamin A, retinal or retinoic acid.

2. Process according to Claim 1, characterised in that 0.8 equivalents of an ether-diol precursor of vitamin A are reacted with 2 equivalents of titanium trichloride and 1 equivalent of lithium aluminium hydride.

3. Process according to either of Claim 1 and 2, characterised in that the ether-diol precursor of vitamin A is reacted at a temperature near 20°C.

4. Process according to one of Claim 1 to 3, characterised in that the anhydrous solvent is an ether such as diethyl ether, dioxane, tetrahydrofuran or mixtures thereof.

5. Process according to one of Claim 1 to 4, characterised in that the ether-diol precursor of vitamin A is chosen from the group consisting of :

— the all-trans ether-diol of formula (VII) :

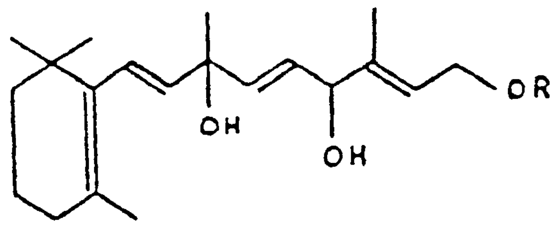

(VII)

in which formula R is a $C_1$-$C_6$ alkyl residue, an aralkyl residue optionally substituted by one or more alkoxy groups such as 4′-methoxybenzyl and 3′, 4′-dimethoxy-benzyl or a

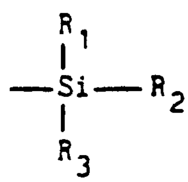

residue in which $R_1$,$R_2$ and $R_3$, which are identical or different, denote a linear or branched $C_1$-$C_6$ alkyl residue or an aryl residue, optionally substituted ;

— the 10-trans ether-diol of formula (VIII′a) :

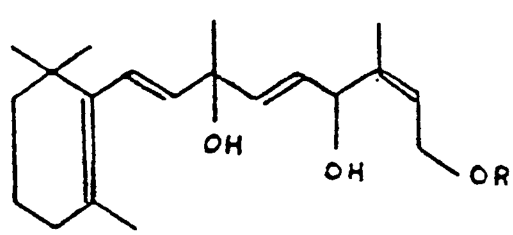

(VIII′a)

and

— the 10-cis ether-diol of formula (VIII′b) :

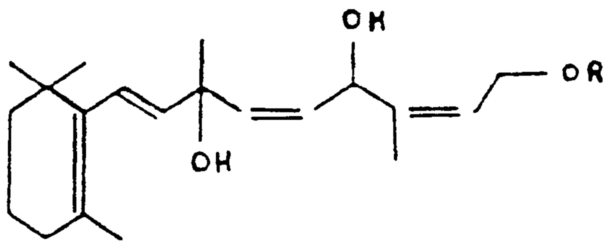

(VIII'b)

in which formulae R has the abovementioned meanings.

6. Process according to Claim 5, characterised in that the all-trans ether-diol precursor of vitamin A, of formula (VII), is obtained by etherification of the corresponding triol of formula (VIa) :

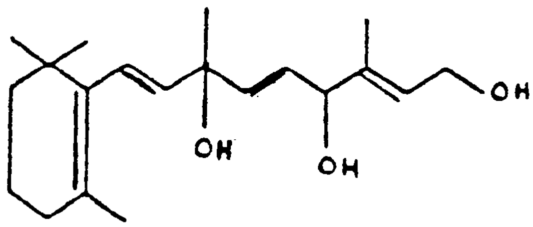

(VIa)

with a $C_1$-$C_6$ alkyl residue, an aralkyl residue optionally substituted by one or more alkoxy groups, especially 4'-methoxy benzyl and 3', 4'-dimethoxybenzyl, or a

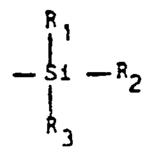

residue where $R_1$, $R_2$ and $R_3$, which are identical or different, denote a branched or linear $C_1$-$C_6$ alkyl residue or an optionally substituted aryl residue, in the presence of imidazole at room temperature.

7. Process according to Claim 6, characterised in that to obtain the compound of formula (VIa) an acetylenic ester of ethynyl-β-ionol of formula (V)

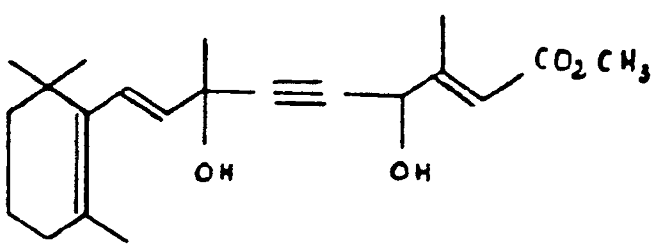

(V)

is reacted with lithium aluminium hydride.

8. Process according to Claim 7, characterised in that to obtain the compound of formula (V) ethynyl-β-ionol of formula (III)

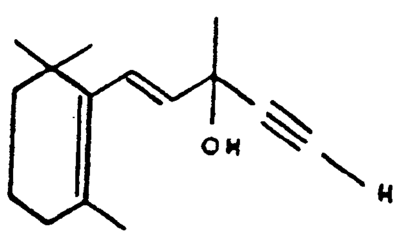

(III)

is reacted with ethylmagnesium bromide and then with methyl γ-oxosenecioate of formula (IV)

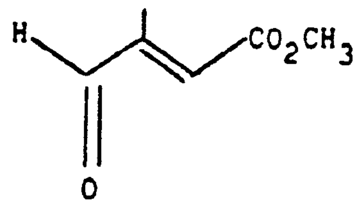

(IV)

9. Process according to Claim 5, characterised in that the 10-cis ether-diol precursor of vitamin A, of formula (VIII'b) is obtained by etherification of the end alcohol functional group of the compound of formula (VIb) :

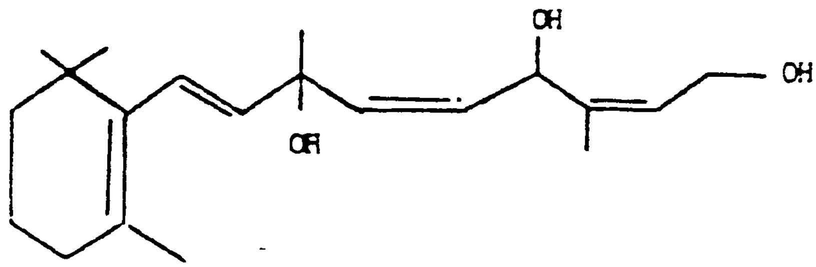

(VIb)

with a $C_1$-$C_6$ alkyl residue, an aralkyl residue optionally substituted by one or more alkoxy groups, especially 4'-methoxybenzyl and 3', 4'-dimethoxybenzyl or of a $SiR_1R_2R_3$ residue where $R_1$, $R_2$ and $R_3$ have the meanings shown in Claim 5, in the presence of imidazole, at room temperature, followed by an extraction with ether.

10. Process according to Claim 9, characterised in that to obtain the compound of formula (VIb) the compound of formula (VI')

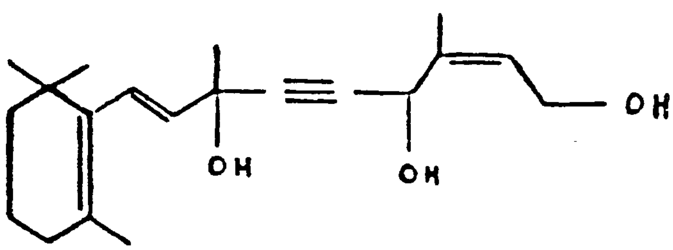

(VI')

is reduced with hydrogen in the presence of the Lindlar catalyst.

11. Process according to Claim 5, characterised in that the 10-cis ether-diol of formula (VIII'b) is obtained by reduction using hydrogen in the presence of the Lindlar catalyst of the compound of formula (VII')

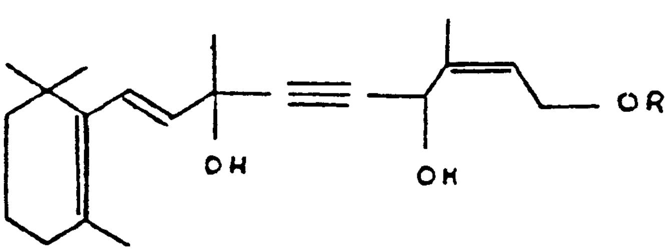

(VII')

in which formula R has the same meanings as in Claim 5.

12. Process according to Claim 5, characterised in that the 10-trans ether-diol of formula (VIII'a) is obtained by reduction with lithium aluminium hydride of the compound of formula (VII')

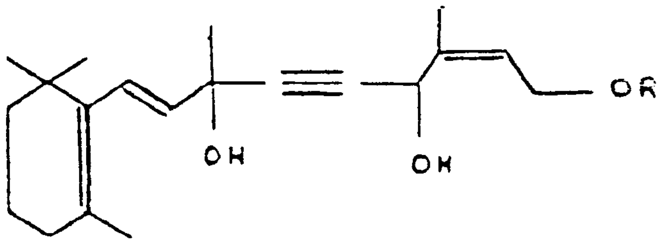

(VII')

in which formula R has the same meanings as in Claim 5 and extraction with ether of the organic phase containing the compound of formula (VIII'a).

13. Process according to either of Claims 11 and 12, characterised in that the compound of formula (VII') is obtained by etherifying the compound of formula (VI')

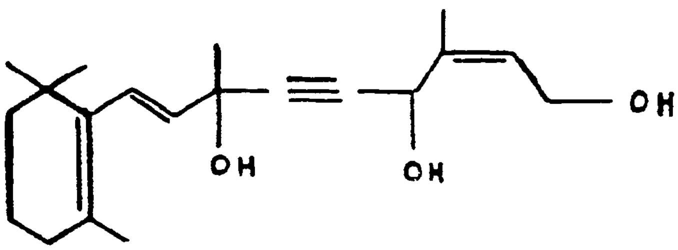

(VI')

a $C_1$-$C_6$ alkyl residue, an aralkyl residue optionally substituted by one or more alkoxy groups, especially the 4'-methoxybenzyl or 3', 4'-dimethoxybenzyl residue, or the

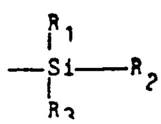

residue, where $R_1$, $R_2$ and $R_3$ have the same meanings as in Claim 5, in the presence of imidazole at room temperature.

14. Process according to Claim 13, characterised in that to obtain the compound of formula (VI') a compound of formula (V') :

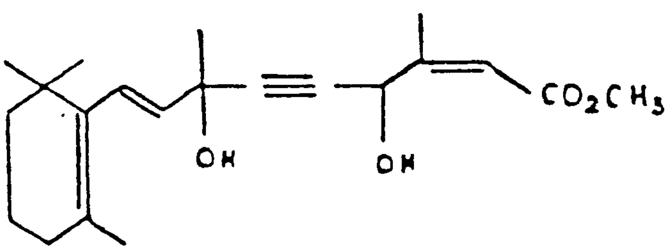

(V')

is reacted with diisobutylaluminium hydride.

15. Process according to Claim 14, characterised in that to obtain the compound of formula (V') ethynyl-β-ionol of formula (III) is reacted with ethylmagnesium bromide and a compound of formula (IV').

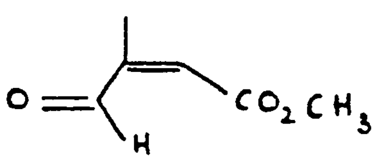

(IV')

16. Process according to Claim 15, characterised in that the compound of formula (IV') is prepared by reacting a butenolide of formula :

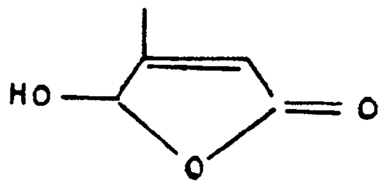

with sodium hydride and then with trimethyloxonium fluoroborate.